(19) Europäisches Patentamt
European Patent Office
Office européen des brevets

(11) Veröffentlichungsnummer: **0 230 967 B1**

# (12) EUROPÄISCHE PATENTSCHRIFT

(45) Veröffentlichungstag der Patentschrift: **23.10.91**

(51) Int. Cl.5: **C07D 225/06**, A61K 31/395, C07D 221/10

(21) Anmeldenummer: **87100733.2**

(22) Anmeldetag: **20.01.87**

## (54) Benzazecin-Derivate.

(30) Priorität: **23.01.86 CH 266/86**

(43) Veröffentlichungstag der Anmeldung: **05.08.87 Patentblatt 87/32**

(45) Bekanntmachung des Hinweises auf die Patenterteilung: **23.10.91 Patentblatt 91/43**

(84) Benannte Vertragsstaaten: **AT BE CH DE ES FR GR IT LI LU NL SE**

(56) Entgegenhaltungen: **US-A- 4 341 786**

(73) Patentinhaber: **F. HOFFMANN-LA ROCHE AG**
**Postfach 3255**
**CH-4002 Basel(CH)**

(72) Erfinder: **Aschwanden, Werner**
**Grellingerstrasse 4**
**CH-4107 Ettingen(CH)**
Erfinder: **Imhof, René, Dr.**
**Bleumatthöhe 3**
**CH-5264 Gipf-Oberfrick(CH)**
Erfinder: **Jakob, Roland, Dr.**
**Oberer Baselblick 26**
**W-7854 Inzlingen(DE)**
Erfinder: **Kyburz, Emilio, Dr.**
**Unterer Rebbergweg 127**
**CH-4153 Reinach(CH)**

(74) Vertreter: **Lederer, Franz, Dr. et al**
**Patentanwalt Dr. Franz Lederer Lucile-Grahn-Strasse 22**
**W-8000 München 80(DE)**

EP 0 230 967 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Benzazecin-Derivate. Im speziellen betrifft sie Benzazecin-Derivate der allgemeinen Formel

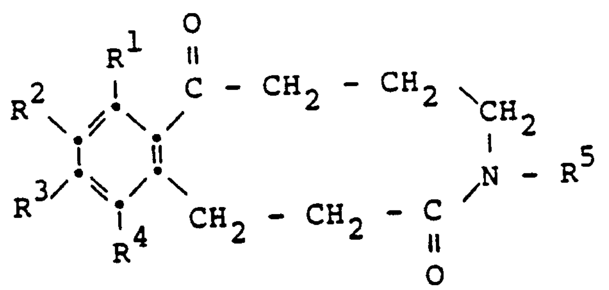

I

worin $R^1$ und $R^2$ je Wasserstoff oder Chlor, $R^3$ Wasserstoff, Fluor, Chlor, Brom oder Methoxy, $R^4$ Wasserstoff, Chlor oder Methoxy und $R^5$ Wasserstoff, Acetyl, Propionyl, Benzoyl, Chlorbenzoyl, Methoxybenzoyl oder Phenylacetyl bedeuten, mit der Massgabe, dass 2 oder 3 der Symbole $R^1$ bis $R^4$ Wasserstoff bedeuten.

Diese Verbindungen sind neu und zeichnen sich durch wertvolle pharmakodynamische Eigenschaften aus.

Gegenstand der vorliegenden Erfindung sind in erster Linie Benzazecin-Derivate der allgemeinen Formel I als solche und als pharmazeutische Wirkstoffe, Verfahren und Zwischenprodukte für die Herstellung dieser Benzazecin -Derivate, ferner diese enthaltende Arzneimittel und die Herstellung solcher Arzneimittel, sowie die Verwendung von Benzazecin-Derivaten der allgemeinen Formel I bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung cognitiver Funktionen und die Verwendung von Benzazecin-Derivaten der allgemeinen Formel I zur Herstellung von Arzneimitteln für die Bekämpfung bzw. Verhütung der cerebralen Insuffizienz bzw. für die Verbesserung cognitiver Funktionen.

Der in der vorliegenden Beschreibung verwendete Ausdruck "nieder" bezeichnet Reste bzw. Verbindungen, welche höchstens 7, vorzugsweise höchstens 4 Kohlenstoffatome enthalten. Der Ausdruck "Alkyl" bezeichnet geradkettige oder verzweigte gesättigte Kohlenwasserstoffreste, wie Methyl, Aethyl, n-Propyl, Isopropyl, n-Butyl, Isobutyl, s-Butyl, t-Butyl und dergleichen. Der Ausdruck "Acyl" bezeichnet Reste, welche sich von organischen Säuren durch Elimination der Hydroxylgruppe ableiten und umfasst demnach z.B. Alkanoylreste (wie Acetyl, Propionyl und dergleichen), Aroylreste (wie Benzoyl, p-Chlorbenzoyl, o-Methoxybenzoyl, m-Methoxybenzoyl, p-Methoxybenzoyl und dergleichen), Aryl-Alkanoylreste (wie Phenylacetyl und dergleichen) usw.

Unter den Benzazecin-Derivaten der allgemeinen Formel I sind diejenigen bevorzugt, worin $R^1$ Chlor und $R^2$, $R^3$ und $R^4$ je Wasserstoff oder $R^2$ Chlor und $R^1$, $R^3$ und $R^4$ je Wasserstoff oder $R^3$ Fluor, Chlor, Brom oder Methoxy und $R^1$, $R^2$ und $R^4$ Wasserstoff oder $R^4$ Chlor oder Methoxy und $R^1$, $R^2$ und $R^3$ je Wasserstoff oder $R^1$ und $R^3$ je Chlor und $R^2$ und $R^4$ je Wasserstoff bedeuten. Die bevorzugte Bedeutungsmöglichkeit des Symbols $R^5$ in Formel I ist Acetyl. Ganz besonders bevorzugte Verbindungen der allgemeinen Formel I sind:

4-Acetyl-9-chlor-1,2,4,5,6,7-hexahydro-4-benzazecin-3,8-dion,
4-Acetyl-11-chlor-1,2,4,5,6,7-hexahydro-4-benzazecin-3,8-dion,
4-Acetyl-11-fluor-1,2,4,5,6,7-hexahydro-4-benzazecin-3,8-dionund
4-Acetyl-9,11-dichlor-1,2,4,5,6,7-hexahydro-4-benzazecin-3,8-dion.

Weitere bevorzugte Verbindungen der allgemeinen Formel I sind:

4-Acetyl-11-methoxy-1,2,4,5,6,7-hexahydro-4-benzazecin-3,8-dion,
11-Chlor-1,2,4,5,6,7-hexahydro-4-propionyl-4-benzazecin-3,8-dion,
4-Benzoyl-11-chlor-1,2,4,5,6,7-hexahydro-4-benzazecin-3,8-dion,
4-(o-Methoxybenzoyl)-11-chlor-1,2,4,5,6,7-hexahydro-4-benzazecin-3,8-dion,
4-(m-Methoxybenzoyl)-11-chlor-1,2,4,5,6,7-hexahydro-4-benzazecin-3,8-dion,
4-(p-Methoxybenzoyl)-11-chlor-1,2,4,5,6,7-hexahydro-4-benzazecin-3,8-dion,
11-Chlor-4-(p-chlorbenzoyl)-1,2,4,5,6,7-hexahydro-4-benzazecin-3,8-dion und
4-Acetyl-1,2,4,5,6,7-hexahydro-12-methoxy-4-benzazecin-3,8-dion.

Repräsentative Beispiele für Verbindungen der Formel I sind auch:
9-Chlor-1,2,4,5,6,7-hexahydro-4-benzazecin-3,8-dion;
4-Acetyl-10-chlor-1,2,4,5,6,7-hexahydro-4-benzazecin-3,8-dion;
11-Chlor-1,2,4,5,6,7-hexahydro-4-(phenylacetyl)-4-benzazecin-3,8-dion;
11-Chlor-1,2,4,5,6,7-hexahydro-4-benzazecin-3,8-dion;
4-Acetyl-11-brom-1,2,4,5,6,7-hexahydro-4-benzazecin-3,8-dion; und
4-Acetyl-12-chlor-1,2,4,5,6,7-hexahydro-4-benzazecin-3,8-dion.

Die Benzazecin-Derivate der allgemeinen Formel I können erfindungsgemäss dadurch hergestellt werden, dass man

a) ein Benzochinolin-Derivat der allgemeinen Formel

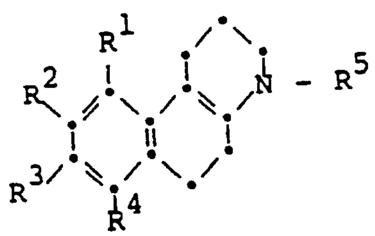

II

worin $R^1$, $R^2$, $R^3$, $R^4$ und $R^5$ die eingangs erwähnte Bedeutung besitzen,
oxydiert oder

b) von einem Benzazecin-Derivat der allgemeinen Formel

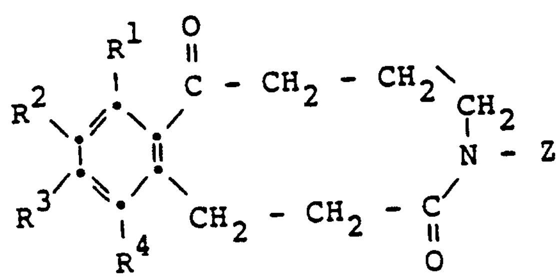

III

worin $R^1$, $R^2$, $R^3$ und $R^4$ die eingangs erwähnte Bedeutung besitzen und Z eine abspaltbare Gruppe bedeutet,

die mit Z bezeichnete Gruppe entfernt.

Die Verbindungen der obigen Formel II sind neu und ebenfalls Gegenstand der vorliegenden Erfindung, ebenso auch ein Verfahren zu ihrer Herstellung, welches dadurch gekennzeichnet ist, dass man

a) ein Benzochinolinon-Derivat der allgemeinen Formel

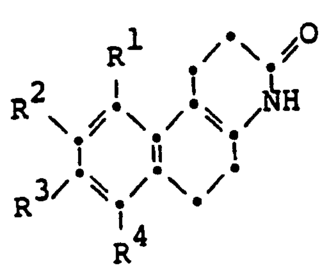

IV

worin $R^1$, $R^2$, $R^3$ und $R^4$ die eingangs erwähnte Bedeutung besitzen,
reduziert oder

b) eine Verbindung der Formel

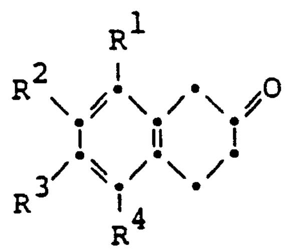

V

worin $R^1$, $R^2$, $R^3$ und $R^4$ die eingangs erwähnte Bedeutung besitzen, in Gegenwart einer starken Base mit einer Verbindung der allgemeinen Formel

$X-(CH_2)_3-NH_2$ VI

worin X eine Abgangsgruppe bedeutet, umsetzt und erwünschtenfalls die erhaltene Verbindung der allgemeinen Formel II, worin $R^5$ Wasserstoff bedeutet, durch Acetyl, Propionyl, Benzoyl, Chlorbenzoyl, Methoxybenzoyl oder Phenylacetyl N-substituiert.

Manche der Benzochinolin-Derivate der allgemeinen Formel II haben ähnliche pharmakodynamische Eigenschaften wie die Benzazecin-Derivate der Formel I, und zwar insbesondere

4-Acetyl-8-chlor-1,2,3,4,5,6-hexahydrobenzo[f]chinolin,

4-Acetyl-10-chlor-1,2,3,4,5,6-hexahydrobenzo[f]chinolin

und

8-Chlor-4-(p-chlorbenzoyl)-1,2,3,4,5,6-hexahydrobenzo[f]chinolin.

Gegenstand der vorliegenden Erfindung sind demnach auch pharmakodynamisch aktive Benzochinolin-Derivate der allgemeinen Formel II als pharmazeutische Wirkstoffe, diese enthaltende Arzneimittel und die Herstellung solcher Arzneimittel, sowie deren Verwendung bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung cognitiver Funktionen und deren Verwendung zur Herstellung von Arzneimitteln für die Bekämpfung bzw. Verhütung der cerebralen Insuffizienz bzw. für die Verbesserung cognitiver Funktionen.

Diejenigen unter den Verbindungen der Formel III, worin Z Acetyl, Propionyl, Benzoyl, Chlorbenzoyl, Methoxybenzoyl oder Phenylacetyl bedeutet, fallen unter den Umfang der Formel I; diejenigen unter den Verbindungen der Formel III, worin Z eine andere Bedeutung besitzt, sind ebenfalls neu und ebenfalls Gegenstand der vorliegenden Erfindung.

Die erfindungsgemässe Oxidation von Benzochinolin-Derivaten der allgemeinen Formel II zu entsprechenden Benzazecin-Derivaten der allgemeinen Formel I erfolgt zweckmässigerweise mittels m-Chlorperbenzoesäure in einem inerten organischen Lösungsmittel, beispielsweise in einem halogenierten Kohlenwasserstoff, wie Chloroform und dergleichen. Die Temperatur ist nicht kritisch, und die Oxidation kann zweckmässigerweise bei Temperaturen von etwa -20°C bis etwa 30°C erfolgen, vorzugsweise zwischen etwa -5°C und etwa Raumtemperatur.

Weiterhin kann diese Oxidation auch zweckmässigerweise mittels Kaliumpermanganat und Natriumperiodat durchgeführt werden, zweckmässigerweise in einem Zweiphasensystem, bestehend aus Wasser und einem damit nicht mischbaren organischen Lösungsmittel, z.B. einem halogenierten Kohlenwasserstoff, wie Methylenchlorid und dergleichen. Dabei wird vorteilhafterweie ein Phasentransferkatalysator beigefügt, insbesondere ein quartäres Ammoniumsalz, wie Benzyltriäthylammoniumchlorid und dergleichen. Wiederum ist die Reaktionstemperatur nicht kritisch und die Oxidation mittels Kaliumpermanganat/Natriumperiodat kann zweckmässigerweise bei Temperaturen zwischen etwa 0°C und etwa 30°C durchgeführt werden, zweckmässigerweise bei etwa Raumtemperatur.

Für die Durchführung der fraglichen Oxidation eignen sich weiterhin Oxidationsmittel bzw. Oxidationssysteme, wie Peressigsäure, Wasserstoffperoxid und Ameisensäure oder p-Toluolsulfonsäure, Chromschwefelsäure, Jones-Reagenz und dergleichen.

Wenn $R^5$ in Formel II Wasserstoff bedeutet, dann erfolgt die Oxidation zum entsprechenden Benzazecin-Derivat der Forml I äusserst leicht, unter Umständen sogar spontan durch Einwirkung des in der Luft vorhandenen Sauerstoffs.

Benzazecin-Derivate der allgemeinen Formel I können erfindungsgemäss auch dadurch erhalten werden, dass man von einem Benzazecin-Derivat der allgemeinen Formel III die mit Z bezeichnete abspaltbare Gruppe entfernt. Als abspaltbare Gruppen eignen sich in erster Linie leicht abspaltbare Acylgruppen, wie

Acetyl und dergleichen.

Die Abspaltung einer Acetylgruppe erfolgt zweckmässigerweise unter alkalischen Bedingungen, beispielsweise mittels Kaliumcarbonat, Natriumhydrogencarbonat und dergleichen in einem Gemisch von Wasser und einem damit mischbaren organischen Lösungsmittel (z.B. einem niederen Alkanol, wie Methanol), mittels eines niederen Alkalimetall-alkoxyds in einem unter den Reaktionsbedingungen inerten organischen Lösungsmittel, zweckmässigerweise im entsprechenden niederen Alkanol, usw..

Die erfindungsgemässe Reduktion eines Benzochinolinon-Derivats der allgemeinen Formel IV zum entsprechenden Benzochinolin-Derivat der allgemeinem Formel II, worin $R^5$ Wasserstoff bedeute, erfolgt zweckmässigerweise mittels eines komplexen Hydrids, wie Lithiumaluminiumhydrid und dergleichen, in einem unter den Reaktionsbedingungen inerten organischen Lösungsmittel, zweckmässigerweise in einem Aether, wie Tetrahydrofuran, Dioxan und dergleichen. Die Reaktionstemperatur ist nicht kritisch, und die fragliche Reduktion kann bei Temperaturen zwischen etwa Raumtemperatur und etwa 120° C erfolgen, zweckmässigerweise bei Rückflusstemperatur.

Die erfindungsgemässe Umsetzung einer Verbindung der Formel V mit einer Verbindung der Formel VI erfolgt in Gegenwart einer starken Base, zweckmässigerweise in Gegenwart einer anorganischen Base, wie Kalium- oder Natriumhydroxid, einer quartären Ammoniumbase, wie Benzyltrimethylammoniumhydroxid u.dgl. Die in Formel VI durch das Symbol X bezeichnete Abgangsgruppe ist zweckmässigerweise ein Halogenatom, insbesondere ein Chloratom, doch kommen auch äquivalente anderer Abgangsgruppen in Betracht, z.B. Alkylsulfonyloxygruppen, wie Mesyloxy, Arylsulfonyloxygruppen, wie Benzolsulfonyloxy, p-Toluolsulfonyloxy, p-Brombenzolsulfonyloxy und dergleichen. Die Verbindung der Formel VI wird zweckmässigerweise in Form eines Säureadditionssalzes eingesetzt, beispielsweise als Hydrochlorid. Die Reaktion erfolgt in Gegenwart eines unter den Reaktionsbedingungen inerten organischen Lösungsmittels, beispielsweise in einem niederen Alkanol, wie Methanol, einem aromatischen Kohlenwasserstoff, wie Toluol, und dergleichen.

Die Reaktionstemperatur ist nicht kritisch, und die Umsetzung der Verbindungen der Formel V und VI kann zweckmässigerweise zwischen Temperaturen von etwa 30° C und etwa 110° C erfolgen, vorzugsweise bei Rückflusstemperatur.

Sowohl bei der Reduktion eines Benzochinolinon-Derivates der allgemeinen Formel IV als auch bei der Umsetzung von Verbindungen der Formel V und VI erhält man entsprechende Verbindungen der allgemeinen Formel II, worin $R^5$ Wasserstoff bedeutet. Diese können erfindungsgemäss entsprechend N-acyliert werden, und zwar zweckmässigerweise in situ, d.h. ohne dass sie gefasst werden. Als Acylierungsmittel verwendet man hierbei den gewünschten Acylrest liefernde reaktionsfähige Derivate der entsprechenden Carbonsäuren, zweckmässigerweise Anhydride, wie Acetanhydrid, Propionsäureanhydrid und dergleichen, Carbonsäurechloride, wie Benzoylchlorid, p-Chlorbenzoylchlorid, o-Methoxybenzoylchlorid, m-Methoxybenzoylchlorid, p-Methoxybenzoylchlorid, Phenylacetylchlorid usw., und dergleichen. In analoger Weise erhält man Verbindungen der Formel III, welche nicht unter den Umfang der allgemeinen Formel I fallen, d.h. worin Z von Acetyl, Propionyl, Benzoyl, Chlorbenzoyl, Methoxybenzoyl oder Phenylacetyl verschieden ist, nämlich indem man entsprechende Verbindungen der allgemeinen Formel II, worin $R^5$ Wasserstoff bedeutet, mit einem die gewünschte Gruppe Z liefernden Mittel behandelt, beispielsweise mittels Benzyloxycarbonylchlorid und dergleichen.

Die Herstellung von Verbindungen der allgemeinen Formeln IV und V erfolgt zweckmässigerweise gemäss dem nachfolgenden Reaktionsschema, worin $R^1$, $R^2$, $R^3$ und $R^4$ die eingangs erwähnte Bedeutung besitzen und $R^6$ und $R^7$ je niederes Alkyl oder zusammen mit dem Stickstoffatom einen heterocyclischen Rest bedeuten, wie Pyrrolin-1-yl, Pyrrolidin-1-yl, Piperidino, Morpholino, 4-(nied.Alkyl)-piperazin-1-yl und dergleichen.

Reaktionsschema

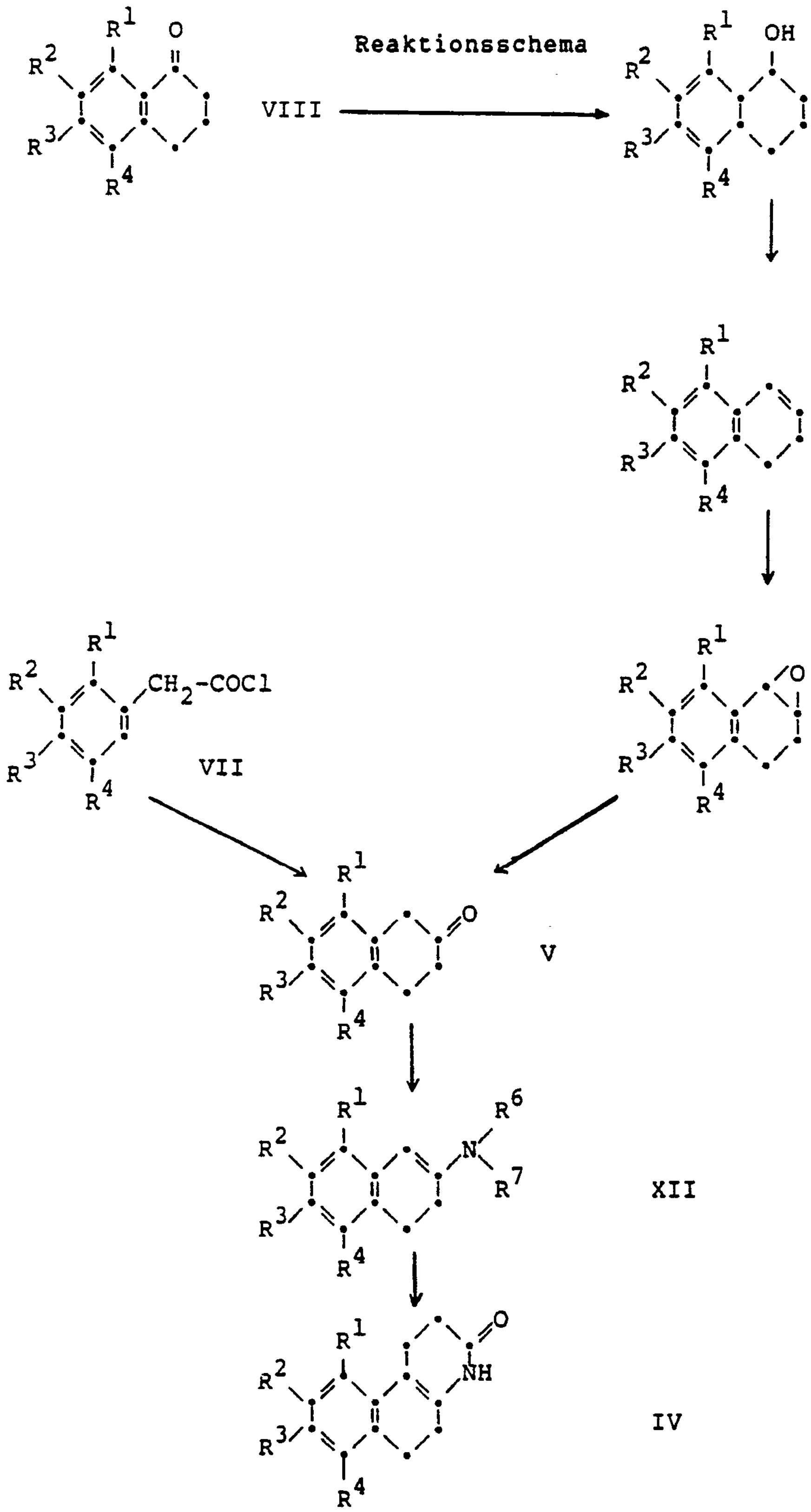

VIII

IX

X

VII

XI

V

XII

IV

Verbindungen der Formel V können in einer Stufe aus Verbindungen der Formel VII hergestellt werden, und zwar durch Umsetzung mit Aethylen in Gegenwart von Aluminiumchlorid oder einer anderen für derartige Reaktionen als Katalysator geeigneten Lewis-Säure. Die Umsetzung erfolgt in Gegenwart eines unter den Reaktionsbedingungen inerten organischen Lösungsmittels, zweckmässigerweise in einem halogenierten Kohlenwasserstoff, wie Methylenchlorid.

Verbindungen der Formel V können aber auch durch eine mehrstufige Synthese ausgehend von Verbindungen der Formel VIII hergestellt werden. Zunächst wird die Verbindung der Formel VIII zur entsprechenden Verbindung der Formel IX reduziert, und zwar zweckmässigerweise mit einem komplexen Hydrid, wie Natriumborhydrid oder dergleichen. Die erhaltene Verbindung der Formel IX wird dann zur entsprechenden Verbindung der Formel X dehydratisiert, und zwar zweckmässigerweise unter sauren Bedingungen, z.B. mittels einer starken Säure, wie p-Toluolsulfonsäure oder dergleichen, in einem mit Wasser nicht mischbaren, jedoch azeotrop destillierenden Lösungsmittel bei Rückflusstemperatur, wobei das entstehende Wasser kontinuierlich entfernt wird. Die Verbindung der Formel X wird dann zur entsprechenden Verbindung der Formel XI oxydiert. Diese Oxydation erfolgt zweckmässigerweise mittels m-Chlorperbenzoesäure oder dergleichen in einem unter den Reaktionsbedingungen inerten organischen Lösungsmittel, beispielsweise in einem chlorierten Kohlenwasserstoff, wie Methylenchlorid. Zu Verbindungen der Formel V gelangt man dann ausgehend von entsprechenden Verbindungen der Formel XI beispielsweise durch Behandlung mit einer ätherischen Lösung von Magnesiumbromid oder durch Behandlung mit einer organischen Sulfonsäure, wie p-Toluolsulfonsäure, o.dgl., in einem inerten organischen Lösungsmittel, wie Toluol o.dgl.

Zur Herstellung einer Verbindung der Formel XII wird eine entsprechende Verbindung der Formel V mit einem sekundären Amin der Formel $HNR^6R^7$ wie z.B. Pyrrolidin, umgesetzt, und zwar in Gegenwart einer Säure, zweckmässigerweise einer organischen Sulfonsäure, wie p-Toluolsulfonsäure oder dergleichen, in einem unter den Reaktionsbedingungen inerten organischen Lösungsmittel, beispielsweise in einem aromatischen Kohlenwasserstoff, wie Benzol; das hierbei entstehende Wasser wird aus dem Reaktionssystem entfernt, beispielsweise durch Zugabe von Molekularsieb oder durch azeotrope Destillation. Zu Verbindungen der Formel IV gelangt man schliesslich dadurch, dass man eine entsprechende Verbindung der Formel XII mit Acrylamid zur Reaktion bringt, und zwar zweckmässigerweise in Gegenwart einer Säure, z.B. einer organischen Sulfonsäure, wie p-Toluolsulfonsäure, eines sauren Ionenaustauschers oder dergleichen, bei Temperaturen von etwa 100°C bis etwa 200°C, zweckmässigerweise von etwa 100-150°C, wobei als Lösungsmittel niedere Alkanole, wie Aethanol, oder dergleichen verwendet werden können.

Wie eingangs erwähnt, sind die Benzacecin-Derivate der Formel I neue Verbindungen mit äusserst wertvollen pharmakodynamischen Eigenschaften, und dasselbe gilt auch für manche der Benzochinolin-Derivate der Formel II. Sie weisen nur eine geringe Toxizität auf, und es hat sich gezeigt, dass sie in dem nachstehend beschriebenen Tierversuch experimentell hervorgerufener cerebraler Insuffizienz entgegenzuwirken vermögen.

Die Test-Apparatur ist eine "Skinner box" mit einem elektrifizierbaren Gitterboden (30 × 40 cm) und einer grauen Plastik-Plattform (15 × 15 × 0.8 cm) in der Ecke vorne rechts. Unerfahrene männliche Ratten (100-120 g) werden einzeln auf die Plattform gebracht. Sobald sie auf den Gitterboden hinabsteigen, erhalten sie einen elektrischen Fuss-Schock (0,8 mA). Die normale Reaktion unerfahrener Ratten ist, daraufhin auf die Plattform zurückzuspringen. Da jedoch die Ratten immer nochmals herabzuklettern versuchen, muss die Fuss-Schock-Prozedur für jedes Tier drei- bis fünfmal wiederholt werden. Nach diesen drei bis fünf Wiederholungen pro Tier haben die Ratten eine sogenannte "passive avoidance response" erlernt. d.h. sie versuchen nicht mehr, auf den Gitterboden hinabzusteigen, weil sie wissen, dass sie bestraft werden.

Unmittelbar anschliessend werden drei Gruppen von je 30 Tieren gebildet. Die erste Gruppe erhält eine Injektion (i.p.) von 0,3 mg/kg Scopolamin sowie destilliertes Wasser (2 ml/kg p.o.). Die zweite Gruppe erhält eine Injektion (i.p. von 0,3 mg/kg Scopolamin und eine orale Dosis der Testsubstanz. Die dritte Gruppe erhält ausschliesslich destilliertes Wasser (p.o.).

2 Stunden später wird jede Ratte einmal auf die Plattform in der "Skinner box" gesetzt. Das Kriterium für die Beurteilung dieses Tests zur Ermittlung einer Präparatwirkung auf das Kurzzeit-Gedächtnis ist, ob das Tier Während 60 Sekunden auf der Plattform bleibt oder nicht (das Ergebnis kann also für jedes Tier nur "ja" oder "nein" lauten). Die statistische Signifikanz der Unterschiede zwischen den bei der ersten und der zweiten Gruppe erhaltenen Resultaten wird mittels des Chi-Quadrat-Tests ermittelt.

70-75% der lediglich mit destilliertem Wasser (p.o.) behandelten Tiere erinnern sich 2-4 Stunden nach Erlernen der "passive avoidance response" noch daran, dass sie auf der Plattform bleiben sollten. Bei 85-92% der mit Scopolamin (0,3 mg/kg i.p.) und destilliertem Wasser (p.o.) behandelten Tiere lässt sich während 3-4 Stunden ein retrograder Effekt auf das Kurzzeitgedächtnis feststellen, d.h. sie haben vergessen, dass sie auf der Plattform bleiben müssen. Eine Substanz, welche cerebraler Insuffizienz entgegenzuwirken vermag, kann die durch die Injektion (i.p.) von 0,3 mg/kg Scopolamin hervorgerufene Blockierung des Kurzzeitgedächtnisses aufheben. Eine Dosis eines Präparats wird dann als aktiv gegen Scopolamin bezeichnet, wenn die Anzahl der positiven Ergebnisse ("ja") von derjenigen mit Scopolamin (0,3 mg/kg i.p.) und nur destilliertem Wasser (p.o.) behandelter Kontroll-Tiere signifikant verschieden ist.

In der nachstehenden Tabelle ist angegeben, bei welchen Dosen bestimmte Verbindungen der Formeln I und II in dem vorstehend beschriebenen Versuch eine signifikante Aktivität zeigen. Die Tabelle enthält ausserdem Angaben für die akute Toxizität ($DL_{50}$ in mg/kg bei einmaliger oraler Verabreichung an Mäuse).

**Tabelle**

| Formel | $R^1$ | $R^2$ | $R^3$ | $R^4$ | $R^5$ | Signifikant wirksame Dosen mg/kg p.o. | DL 50 mg/kg p.o. |
|---|---|---|---|---|---|---|---|
| I | Cl | H | H | H | $COCH_3$ | 0,1<br>0,3<br>1<br>3<br>10<br>30 | >5000 |
| I | H | H | Cl | H | $COCH_3$ | 0,001<br>0,003<br>0,01<br>0,03 | >5000 |
| I | H | H | F | H | $COCH_3$ | 0,03<br>0,1<br>0,3<br>1<br>3<br>10 | >5000 |
| I | Cl | H | Cl | H | $COCH_3$ | 0,03<br>0,1<br>0,3<br>1 | >5000 |
| II | H | H | Cl | H | $COCH_3$ | 0,01<br>0,03<br>0,1<br>0,3 | 2500-5000 |
| II | Cl | H | H | H | $COCH_3$ | 0,3<br>1<br>3<br>10 | |

Die Verbindungen der Formel I und pharmakodynamisch wirksame Verbindungen der Formel II können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Wie weiter vorne erwähnt, sind Arzneimittel, enthaltend eine Verbindung der Formel I und/oder eine pharmakodynamisch wirksame Verbindung der Formel II ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I und/oder pharmakodynamisch wirksame Verbindungen der Formel II und erwünschtenfalls einen oder mehrere andere therapeutisch wirksame Stoffe

zusammen mit einem oder mehreren therapeutisch inerten Exzipientien in eine galenische Darreichungsform bringt.

Zur Herstellung von Tabletten, Lacktabletten, Dragées und Hartgelatinekapseln kann man als Excipientien z.B. Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und/oder pharmakodynamisch wirksame Verbindungen der Formel II bei der Bekämpfung bzw. Verhütung der cerebralen Insuffizienz bzw. bei der Verbesserung cognitiver Funktionen (wie Erinnerungsvermögen, Lernfähigkeit, Interesse an der Umwelt und Selbstsorge) verwenden, beispielsweise in der Geriatrie, bei Intoxikationen, wie Alkoholismus, und bei cerebro-vaskulären Störungen; mögliche weitere Einsatzgebiete sind vestibuläre Störungen (wie Menière-Krankheit) und Entwicklungsstörungen (wie Dyslexie). Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2500 mg angemessen sein, wobei aber die soeben angegebene obere Grenze auch überschritten werden kann, wenn sich dies als angezeigt erweisen sollte.

Gegenstand der Erfindung ist schliesslich auch die Verwendung der Verbindungen der Formel I und/oder von pharmakodynamisch wirksamen Verbindungen der Formel II zur Herstellung von Arzneimitteln für die Bekämpfung bzw. Verhütung der cerebralen Insuffizienz bzw. für die Verbesserung cognitiver Funktionen.

In den nachfolgenden Beispielen, welche die vorliegende Erfindung illustrieren, deren Umfang jedoch in keiner Weise einschränken sollen, sind sämtliche Temperaturen in Celsiusgraden angegeben.

Beispiel 1

a) Zu 218 g Aluminiumchlorid in 1000 ml Methylenchlorid werden unter Rühren zwischen 0° und 5° innerhalb von 1 Stunde 154,8 g 2-Chlor-phenylacetylchlorid, gelöst in 290 ml Methylenchlorid, zugetropft. Danach wird in das Gemisch zwischen 0° und 5° während 40 Minuten Aethylen eingeleitet, worauf das Reaktionsgemisch 1 Stunde bei Raumtemperatur gerührt und dann zwischen 0° und 5° mit 570 ml kaltem Wasser versetzt wird. Die Methylenchloridphase wird mit 2 × 500 ml 2N Salzsäure, 2 × 500 ml Natriumhydrogencarbonatlösung und 700 ml Wasser gewaschen. Die Wasserphasen werden mit 300 ml Methylenchlorid ausgezogen. Die vereinigten Methylenchloridphasen werden über Natriumsulfat getrocknet und im Vakuum eingeengt. Das aus 400 ml tiefsiedendem Petroläther kristallisierte 8-Chlor-3,4-dihydro-2(1H)-naphthalinon zeigt einen Schmelzpunkt von 56-59°.

b) 70,0 g 8-Chlor-3,4-dihydro-2(1H)-naphthalinon werden in 550 ml Benzol und 33 ml Pyrrolidin in Gegenwart von 1,4 g p-Toluolsulfonsäure 2,5 Stunden am Rückfluss gekocht. Das erhaltene rohe 1-(8-Chlor-3,4-dihydro-2-naphthyl)-pyrrolidin wird ohne Reinigung weiterverarbeitet.

c) Zu 89,4 g rohem 1-(8-Chlor-3,4-dihydro-2-naphthyl)-pyrrolidin werden 56,0 g Acrylamid und 3,0 g wasserfreie p-Toluolsulfonsäure zugegeben. Man erhitzt unter Stickstoff je 2 Stunden auf 100° und auf 150°. Das Reaktionsgemisch wird zwischen 300 ml Methylenchlorid und 200 ml Wasser verteilt. Die organische Phase wird mit Wasser gewaschen und durch 500 g Kieselgel (Korngrösse 0,2-0,5 mm) filtriert. Das mit Essigester eluierte 10-Chlor-1,4,5,6-tetrahydrobenzo[h]-chinolin-3(2H)-on zeigt nach Umkristallisieren aus Essigester einen Schmelzpunkt von 186-187°.

d) Zu einer unter Stickstoff gerührten Suspension von 6,49 g Lithiumaluminiumhydrid in 240 ml trockenem Tetrahydrofuran werden innerhalb von 35 Minuten zwischen 20° und 25° portionenweise 20,0 g 10-Chlor-1,4,5,6-tetrahydrobenzo[h]chinolin-3(2H)-on zugegeben. Das Reaktionsgemisch wird anschliessend während 150 Minuten unter Rückfluss zum Sieden erhitzt, dann abgekühlt und hierauf zwischen 0° und 10° mit 21,0 ml 6,5N Natronlauge versetzt. Die entstandene Suspension wird filtriert,

worauf man den Filterrückstand mehrmals mit je 20 ml Tetrahydrofuran nachwäscht und das Filtrat im Vakuum eindampft. Das so erhaltene 10-Chlor-1,2,3,4,5,6-hexahydrobenzo[f]chinolin wird direkt weiterverarbeitet.

e) 20,1 g rohes 10-Chlor-1,2,3,4,5,6-hexahydrobenzo[f]chinolin werden in 40 ml Pyridin und 36 ml Essigsäureanhydrid aufgenommen. Man lässt das Reaktionsgemisch 20 Stunden bei Raumtemperatur stehen, dampft ein, nimmt den verbleibenden Rückstand zweimal in je 150 ml trockenem Toluol auf und dampft die erhaltenen Lösungen zur Trockene ein. Der Rückstand wird in Methylenchlorid gelöst und an 150 g Kieselgel (Korngrösse 0,2-0,5 mm) chromatographiert. Das mit Chloroform eluierte 4-Acetyl-10-chlor-1,2,3,4,5,6-hexahydrobenzo[f]chinolin zeigt nach Umkristallisieren aus Isopropyläther einen Schmelzpunkt von 117-118°.

f) 8,5 g 4-Acetyl-10-chlor-1,2,3,4,5,6-hexahydrobenzo[f]chinolin werden in 205 ml Chloroform gelöst, worauf bei 0° bis +5° 16 g 85%ige m-Chlorperbenzoesäure, gelöst in 205 ml Chloroform, tropfenweise zugegeben werden. Nach 4-stündigem Rühren bei Raumtemperatur werden zum Reaktionsgemisch 4 g Kaliumjodid und 70 ml Wasser zugegeben, worauf man bis zur Entfärbung mit Natriumthiosulfat versetzt. Die Chloroformphase wird mit 70 ml 2N Natronlauge und 2 × 170 ml Wasser gewaschen. Die Wasserphasen werden mit 170 ml Chloroform ausgeschüttelt. Die vereinigten Chloroformextrakte werden getrocknet und im Vakuum eingedampft. Der Rückstand wird in Methylenchlorid gelöst und an 100 g Kieselgel (Korngrösse 0,2-0,5 mm) chromatographiert. Das mit Methylenchlorid eluierte 4-Acetyl-9-chlor-1,2,4,5,6,7-hexahydro-4-benzazecin-3,8-dion zeigt nach Umkristallisieren aus Isopropyläther einen Schmelzpunkt von 115-116°.

Beispiel 2

7,60 g 4-Acetyl-9-chlor-1,2,4,5,6,7-hexahydro-4-benzazecin-3,8-dionwerden in 240 ml Methanol gelöst und mit einem Gemisch von 36 ml Methanol und 4 ml gesättigter Kaliumcarbonatlösung versetzt. Man rührt das Gemisch 1 Stunde bei Raumtemperatur und entfernt danach die Lösungsmittel im Vakuum. Der Rückstand wird in Essigsäureäthylester verrührt; das auskristallisierte 9-Chlor-1,2,4,5,6,7-hexahydro-4-benzazecin-3,8-dion wird abfiltriert und zeigt einen Schmelzpunkt von 211-214°. Das Filtrat wird an 100 g Kieselgel (Korngrösse 0,2-0,5 mm) chromatographiert. Aus dem Essigester-Eluat kann eine weitere Portion 9-Chlor-1,2,4,5,6,7-hexahydro-4-benzazecin-3,8-dion isoliert werden.

Beispiel 3

5,0 g 8-Chlor-3,4-dihydro-2(1H)-naphthalinon, 115 ml Benzol und 17 ml Triton-B (40%ig in Methanol) werden am Rückfluss gekocht, worauf man 5,0 g 3-Chlorpropylamin-hydrochlorid, gelöst in 25 ml Methanol, zutropft und weitere 5 Stunden am Rückfluss erhitzt. Dann werden 5 g Molekularsieb 4A zugegeben, worauf weitere 20 Stunden am Rückfluss gekocht wird. Das Reaktionsgemisch wird nach Filtration im Wasserstrahlvakuum eingeengt, worauf man den Rückstand zwischen Diäthyläther und 3N Salzsäure verteilt und die organische Phase noch zweimal mit 3N Salzsäure extrahiert. Die Salzsäure-Phasen werden mit 2N Natronlauge basisch gestellt und mit Essigester extrahiert. Die Essigesterphasen werden mit Natriumchloridlösung gewaschen, getrocknet und im Vakuum eingedampft. Der Rückstand wird durch 300 g Kieselgel (Korngrösse 0,2-0,5 mm) chromatographiert. Das mit Methylenchlorid-Essigester (Gemisch 1:1) und Essigester eluierte 9-Chlor-1,2,4,5,6,7-hexahydro-4-benzazecin-3,8-dionzeigt nach Verrühren in Diäthyläther einen Schmelzpunkt von 196-198°. Nach Umkristallisation aus Essigester zeigt das Produkt einen Schmelzpunkt von 207-208°.

Im obigen Beispiel wird zunächst 10-Chlor-1,4,5,6-tetrahydrobenzo[h]chinolin-3(2H)-on gebildet, welches nicht isoliert wird, sondern durch Luftoxidation in 9-Chlor-1,2,4,5,6,7-hexahydro-4-benzazecin-3,8-dion übergeht.

Beispiel 4

a) 11,0 g 7-Chlor-3,4-dihydro-2(1H)-naphthalinon wird in 200 ml Benzol und 6,0 ml Pyrrolidin in Gegenwart von 0,25 g p-Toluolsulfonsäure 2 Stunden am Rückfluss gekocht. Das erhaltene 1-(7-Chlor-3,4-dihydro-2-naphthyl)pyrrolidin wird aus 150 ml Isopropyläther umkristallisiert und zeigt einen Schmelzpunkt von 115-116°.

b) Zu 10,6 g 1-(7-Chlor-3,4-dihydro-2-naphthy)pyrrolidin werden 6,45 g Acrylamid zugegeben. Man erhitzt unter Stickstoff je 2 Stunden auf 100° und 150°. Das Reaktionsgemisch wird mit 100 ml Essigsäureäthylester versetzt, worauf man bei Raumtemperatur rührt und das 9-Chlor-1,4,5,6-tetrahydrobenzo[f]chinolin-3(2H)-on abfiltriert, welches nach Umkristallisieren aus Essigsäureäthylester bei 265-267° schmilzt.

c) Zu einer bei 20° unter Stickstoff gerührten Suspension von 1,59 g Lithiumaluminiumhydrid in 60 ml trockenem Tetrahydrofuran werden über einen Zeitraum von 35 Minuten zwischen 20° und 25°, 4,9 g 9-Chlor-1,4,5,6-tetrahydrobenzo[f]chinolin-3(2H)-on portionenweise zugegeben. Das Reaktionsgemisch wird anschliessend während 150 Minuten am Rückfluss gekocht, dann abgekühlt und hierauf zwischen 0° und 10° mit 5,20 ml 6,5N Natronlauge versetzt. Die entstandene Suspension wird filtriert, worauf man den Filterrückstand mehrmals mit je 20 ml Tetrahydrofuran nachwäscht und das Filtrat im Vakuum eindampft. Das so erhaltene 9-Chlor-benzo[f]chinolin wird als Rohprodukt in 10 ml Pyridin und 9,0 ml Essigsäureanhydrid aufgenommen. Man lässt das Reaktionsgemisch 20 Stunden bei Raumtemperatur stehen, dampft dann ein, nimmt den verbleibenden Rückstand zweimal in je 50 ml trockenem Toluol auf und dampft die erhaltenen Lösungen zur Trocken ein. Der Rückstand wird in Methylenchlorid gelöst und an 150 g Kieselgel (Korngrösse 0,2-0,5 mm) chromatographiert. Das mit Methylenchlorid eluierte 4-Acetyl-9-chlorbenzo[f]chinolin zeigt nach Umkristallisieren aus Isopropyläther einen Schmelzpunkt von 151,5-152°.

d) 2,5 g 4-Acetyl-9-chlorbenzo[f]chinolin werden in 60 ml Chloroform gelöst, worauf bei 0° bis +5° 5,02 g 85%ige m-Chlorperbenzoesäure, gelöst in 60 ml Chloroform, tropfenweise zugegeben werden. Danach wird bei Raumtemperatur 3 Stunden nachgerührt. Zum Reaktionsgemisch werden 0,4 g Kaliumjodid und 40 ml Wasser zugegeben, worauf bis zur Entfärbung mit Natriumthiosulfat versetzt wird. Die Chloroformphase wird abgetrennt und mit 40 ml 2N Natronlauge und dann mit 2 × 50 ml Wasser gewaschen. Die Wasserphasen werden mit 100 ml Chloroform ausgeschüttelt. Die vereinigten Chloroformextrakte werden getrocknet und im Vakuum eingedampft. Das aus Isopropyläther umkristallisierte 4-Acetyl-10-chlor-1,2,4,5,6,7-hexahydro-4-benzazecin-3,8-dion zeigt einen Schmelzpunkt von 136-137°.

Beispiel 5

a) Zu 230 g Aluminiumchlorid in 1050 ml Methylenchlorid werden under Rühren zwischen 0° und 5° innerhalb von 60 Minuten 149,4 g 4-Fluorphenylessigsäurechlorid, gelöst in 300 ml Methylenchlorid, zugetropft. Danach wird in das Gemisch zwischen 0-5° während 30 Minuten Aethylen eingeleitet, worauf 1 Stunde bei Raumtemperatur weitergerührt und dann zwischen 0° und 5° innerhalb von 30 Minuten mit 600 ml Eiswasser versetzt wird. Die Methylenchloridphase wird mit 2N Salzsäure, Wasser und gesättigter Natriumhydrogencarbonatlösung gewaschen, über Natriumsulfat getrocknet und eingedampft. Man versetzt den Rückstand mit 250 ml tiefsiedendem Petroläther, lässt über Nacht im Eisschrank stehen und filtriert das 6-Fluor-3,4-dihydro-2(1H)-naphthalinon vom Schmelzpunkt 50-60° ab.

b) 16,7 g 6-Fluor-3,4-dihydro-2(1H)-naphthalinon werden in 200 ml Benzol und 8,4 ml Pyrrolidin in Gegenwart von 0,35 g wasserfreier p-Toluolsulfonsäure 2,5 Stunden am Rückfluss gekocht. Das erhaltene rohe 1-(6-Fluor-3,4-dihydro-2-naphthyl)pyrrolidin wird ohne weitere Reinigung mit 10,8 g Acrylamid und 0,5 g p-Toluolsulfonsäure versetzt. Man erhitzt unter Stickstoff je 2 Stunden auf 100° und 150°. Das Reaktionsgemisch wird in 180 ml Chloroform gelöst und mit Wasser gewaschen. Die organische Phase wird unter Eluieren mit Chloroform über 150 g Kieselgel (Korngrösse 0,2-0,5 mm) filtriert. Nach Umkristallisieren aus Essigsäureäthylester erhält man 8-Fluor-1,4,5,6-tetrahydrobenzo[h]-chinolin-3(2H)-on vom Schmelzpunkt 223-224°.

c) Zu einer bei 20° unter Stickstoff gerührten Suspension von 2,17 g Lithiumaluminiumhydrid in 60 ml trockenem Tetrahydrofuran werden innerhalb von 35 Minuten zwischen 20° und 25° portionenweise 6,2 g 8-Fluor-1,4,5,6,-tetrahydrobenzo[h]chinolin-3(2H)-on zugegeben. Das Reaktionsgemisch wird anschliessend während 150 Minuten unter Rückfluss zum Sieden erhitzt, dann abgekühlt und hierauf zwischen 0° und 10° mit 7,0 ml 6,5N Natronlauge versetzt. Die entstandene Suspension wird filtriert, worauf man den Filterrückstand mehrmals mit je 20 ml Tetrahydrofuran nachwäscht und das Filtrat im Vakuum eindampft. Das so erhaltene 8-Fluor-1,2,3,4,5,6-hexahydrobenzo[f]chinolin wird direkt weiterverarbeitet.

d) 6 g rohes 8-Fluor-1,2,3,4,5,6-hexahydrobenzo[f]chinolin werden in 13 ml Pyridin und 12 ml Essigsäureanhydrid aufgenommen. Man lässt das Reaktionsgemisch 20 Stunden bei Raumtemperatur stehen, dampft dann ein, nimmt den verbleibenden Rückstand zweimal in je 50 ml trockenem Toluol auf und dampft die erhaltenen Lösungen zur Trockene ein. Der Rückstand wird in Methylenchlorid gelöst und an 150 g Kieselgel (Korngrösse 0,2-0,5 mm) chromatographiert. Das mit Methylenchlorid eluierte 4-Acetyl-8-fluor-1,2,3,4,5,6-hexahydrobenzo[f]chinolin zeigt nach Umkristallisieren aus Isopropyläther einen Schmelzpunkt von 101-102°.

e) 2,48 g 4-Acetyl-8-fluor-1,2,3,4,5,6-hexahydrobenzo[f]chinolin werden in 60 ml Chloroform gelöst, worauf bei 0° bis +5° 5,25 g 85%ige m-Chlorperbenzoesäure, gelöst in 40 ml Chloroform, tropfenweise

zugegeben werden. Danach rührt man 3 Stunden bei Raumtemperatur, gibt 1,10 g Kaliumjodid und 15 ml Wasser zu und versetzt bis zur Entfärbung mit Natriumthiosulfat. Die Chloroformphase wird abgetrennt und mit 15 ml 2N Natronlauge und 2 x 40 ml Wasser gewaschen. Die wässerigen Auszüge werden mit 20 ml Chloroform ausgeschüttelt. Die vereinigten Chloroformextrakte werden getrocknet und im Vakuum eingedampft. Das aus Essigester umkristallisierte 4-Acetyl-11-fluor-1,2,4,5,6,7-hexahydro-4-benzazecin-3,8-dion zeigt einen Schmelzpunkt von 161-162°.

Beispiel 6

a) Man löst 1155 g (6,4 Mol) 6-Chlor-3,4-dihydro-2(1H)-naphthalinon in 5 l Toluol, tropft 500 g (7,0 Mol) Pyrrolidin und anschliessend eine Lösung von 26 g (0,14 Mol) p-Toluolsulfonsäure in Toluol zu und kocht unter Rückfluss. Wenn ca. 120 ml Wasser abgeschieden sind, destilliert man 4 l Toluol ab und lässt langsam abkühlen. Dabei kristallisiert ein Festkörper aus. Filtrieren und Waschen mit Aceton ergibt 1-(6-Chlor-3,4-dihydro-2-naphthyl)pyrrolidin vom Schmelzpunkt 117-118°.
Einengen der Mutterlauge, Suspendieren des Rückstandes in Aether, Filtrieren und Waschen mit Aceton ergibt eine weitere Portion des obigen Produkts vom Schmelzpunkt 117-118°.
b) Man kocht 701 g (3 Mol) 1-(6-Chlor-3,4-Dihydro-2-naphthyl)pyrrolidinund 640 g (19 Mol) Acrylamid in 7 l Aethanol unter Zusatz von 70 g Amberlit IR200 3 Tage lang unter Rückfluss, filtriert den ausgefallenen Feststoff ab, kristallisiert extraktiv mit Dioxan und erhält 8-Chlor-1,4,5,6-tetrahydrobenzo[f]chinolin-3-(2H)-on vom Schmelzpunkt 228-230°C.
c) Man suspendiert unter Argon 147 g (1,94 Mol) Lithiumaluminiumhydrid in 4 l Tetrahydrofuran, gibt langsam 454 g (1,94 Mol) 8-Chlor-1,4,5,6-tetrahydrobenzo[f]chinolin-3(2H)-on zu und kocht 2,5 Stunden unter Rückfluss. Dann kühlt man ab, tropft 470 ml 18-proz. Natronlauge zu, rührt 30 Minuten bei Raumtemperatur, filtriert und wäscht den Filterrückstand mit Tetrahydrofuran. Beim Eindampfen des Filtrats erhält man 8-Chlor-1,2,3,4,5,6-hexahydrobenzo[f]chinolin als gelbes Oel.
d) Man löst 229 g (1,04 Mol) 8-Chlor-1,2,3,4,5,6-hexahydrobenzo[f]chinolinin 2 l Methylenchlorid, versetzt mit 115 g (1,14 Mol) Triäthylamin und tropft bei 0° 89,5 g (1,14 Mol) Acetylchlorid in 400 ml Methylenchlorid zu. Nach 1-stündigem Rühren bei Raumtemperatur giesst man das Gemisch in Wasser, extrahiert mit Methylenchlorid und trocknet die Methylenchloridphase mit Magnesiumsulfat. Abdestillieren des Lösungsmittels im Vakuum ergibt ein Rohprodukt, das man in 500 ml Aether suspendiert und abfiltriert. Man erhält 4-Acetyl-8-chlor-1,2,3,4,5,6-hexahydrobenzo[f]chinolin vom Schmelzpunkt 106-108°. Einengen der Mutterlauge und Chromatographieren (Kieselgel/Chloroform) ergibt eine weitere Portion vom Schmelzpunkt 106-108°.

4-Acetyl-8-chlor-1,2,3,4,5,6-hexahydrobenzo[f]chinolin kann aus 6-Chlor-3,4-dihydro-2(1H)-naphthalinon auch wie folgt hergestellt werden:

Unter Argon löst man 100 g 6-Chlor-3,4-dihydro-2(1H)-naphthalinon (0,55 Mol) in 2 l Toluol, versetzt mit 64,0 g pulverisiertem Kaliumhydroxid, erhitzt auf Siedetemperatur, gibt während 30 Minuten portionsweise 165 g 3-Chlorpropylaminhydrochlorid zu und kocht am Wasserabscheider bis im Dünnschichtchromatogramm kein Edukt mehr nachzuweisen ist. Nach Abkühlen auf Raumtemperatur versetzt man mit 155 ml Triäthylamin. Unter Eiskühlung, so dass ein Innentemperatur von 25° nicht überschritten wird, versetzt man tropfenweise 60 ml Acetylchlorid, gelöst in 450 ml Toluol. Man rührt 1 Stunde bei Raumtemperatur, extrahiert mit Wasser/Methylenchlorid, trocknet mit Magnesiumsulfat, destilliert das Lösungsmittel im Vakuum ab und erhält 4-Acetyl-8-chlor-1,2,3,4,5,6-hexahydrobenzo[f]-chinolin in Form brauner Kristalle, die als Rohprodukt für die nachstehend beschriebene Reaktion eingesetzt werden können.
e) Man löst 115 g (0,44 Mol) 4-Acetyl-8-chlor-1,2,3,4,5,6-hexahydrobenzo[f]chinolin in 1 l Methylenchlorid und tropft bei 0° eine Suspension von 189 g (0,93 Mol) m-Chlorperbenzoesäure (85%ig) in 500 ml Methylenchlorid zu. Nach 1-stündigem Rühren bei Raumtemperatur filtriert man vom gebildeten Niederschlag ab und extrahiert mit 2N Natronlauge und mit Wasser. Trocknen der organischen Phase mit Magnesiumsulfat, Abdestillieren des Lösungsmittels im Vakuum und Umkristallisieren des Rückstands aus Essigester-Aether ergibt 4-Acetyl-11-chlor-1,2,4,5,6,7-hexahydro-4-benzazecin-3,8-dion als weisse Kristalle vom Schmelzpunkt 154-156°.

Beispiel 7

a) Man löst 8,40 g (0,038 Mol) 8-Chlor-1,2,3,4,5,6,-hexahydrobenzo[f]chinolin in 20 ml Pyridin und tropft bei 0° 23,4 g (0,172 Mol) Propionsäureanhydrid zu. Nach Rühren über Nacht bei Raumtemperatur extrahiert man mit Wasser und Essigester, trocknet mit Magnesumsulfat und destilliert das Lösungsmittel

im Vakuum ab. Chromatographieren (Kieselgel, Chloroform-Hexan 3:1) und Kristallisieren (Essigester) ergibt 4-Propionyl-8-chlor-1,2,3,4,5,6-hexahydrobenzo[f]chinolin als weisse Kristalle vom Schmelzpunkt 115-117°.

b)Man löst 7,85 g (0,028 Mol) 4-Propionyl-8-chlor-1,2,3,4;5,6-hexahydrobenzo[f]chinolin in 80 ml Chloroform und tropft bei 0° eine Suspension von 11,6 g (0,058 Mol) 85%ige m-Chlorperbenzoesäure in 100 ml Chloroform zu. Nach 3-stündigem Rühren bei Raumtemperatur extrahiert man mit 2N Natronlauge und Wasser, trocknet mit Magnesiumsulfat und destilliert das Lösungsmittel im Vakuum ab. Kristallisation aus Essigester-Aether ergibt 11-Clor-1,2,4,5,6,7-hexahydro-4-propionyl -4-benzazecin-3,8-dion als weisse Kristalle vom Schmelzpunkt 149-151°.

Beispiel 8

a) Man löst 7,00 g (0,032 Mol) 8-Chlor-1,2,3,4,5,6-hexahydrobenzo[f]chinolin in 50 ml Pyridin und tropft bei 0° 6,75 g (0,048 Mol) Benzoylchlorid zu. Nach Rühren über Nacht bei Raumtemperatur extrahiert man mit Wasser und Chloroform, trocknet mit Magnesiumsulfat und destilliert das Lösungsmittel im Vakuum ab. Chromatographieren (Kieselgel, Chloroform-Hexan, 1:1) und Kristallisieren (Chloroform-Hexan) ergibt 4-Benzoyl-8-chlor-1,2,3,4,5,6-hexahydrobenzo[f]chinolin als weisse Kristalle vom Schmelzpunkt 194-196°.

b) Man löst 7,80 g (0,024 Mol) 4-Benzoyl-8-chlor-1,2,3,4,5,6-hexahydrobenzo[f]chinolin in 100 ml Chloroform und tropft bei 0° eine Suspension von 9,80 g (0,05 Mol) 85%ige m-Chlorperbenzoesäure in 100 ml Chloroform zu. Nach 3-stündigem Rühren bei Raumtemperatur extrahiert man mit 2N Natronlauge und Wasser, trocknet mit Magnesiumsulfat und destilliert das Lösungsmittel im Vakuum ab. Chromatographieren (Kieselgel, Chloroform-Hexan, 1:2) und Kristallisieren (Essigester-Hexan) ergibt 4-Benzoyl-11-chlor-1,2,4,-5,6,7-hexahydro-4-benzazecin-3,8-dion als weisse Kristalle vom Schmelzpunkt 129-131°.

Beispiel 9

a) Man löst 7,00 g (0,032 Mol) 8-Chlor-1,2,3,4,5,6-hexahydrobenzo[f]chinolin in 50 ml Pyridin und tropft bei 0° 7,45 g (0,048 Mol) Phenylacetylchlorid zu. Nach Rühren über Nacht bei Raumtemperatur giesst man auf Wasser, extrahiert mit Essigester, trocknet mit Magnesiumsulfat und destilliert das Lösungsmittel im Vakuum ab. Chromatographieren (Kieselgel, Chloroform-Hexan 1:1) ergibt 8-Chlor-4-(phenylacetyl)-1,2,3,4,5,6-Hexahydrobenzo[f]chinolin in Form eines Oels.

b) Man löst 7,10 g (0,021 Mol) 8-Chlor-4-(phenylacetyl)-1,2,3,4,5,6-hexahydrobenzo[f]chinolin in 100 ml Chloroform und tropft bei 0° eine Suspension von 8.55 g (0,043 Mol) 85%ige m-Chlorperbenzoesäure in 100 ml Chloroform zu. Nach 3-stündigem Rühren bei Raumtemperatur extrahiert man mit 2N Natronlauge und Wasser, trocknet mit Magnesiumsulfat und destilliert das Lösungsmittel im Vakuum ab. Chromatographieren (Kieselgel, Chloroform-Hexan 1:1) und Kristallisieren (Essigester-Hexan) ergibt 11-Chlor-1,2,4,5,6,7-hexahydro-4-(phenylacetyl)-4-benzazecin -3,8-dion als weisse Kristalle vom Schmelzpunkt 178-180°.

Beispiel 10

Zu einer Lösung von 4,75 g (0,03 Mol) Kaliumpermanganat und 514 g (2,4 Mol) Natriumperiodat in 12 l Wasser gibt man 26,3 g (0,12 Mol) 8-Chlor-1,2,3,4,5,6-hexahydrobenzo[f]chinolin, gelöst in 3 l Methylenchlorid, fügt 3,00 g (0,013 Mol) Benzyltriäthylammoniumchlorid zu und rührt über Nacht bei Raumtemperatur. Man filtriert über Dicalit und extrahiert mit Methylenchlorid. Trocknen mit Natriumsulfat, Abdestillieren des Lösungsmittels im Vakuum und Chromatographieren (Kieselgel, Essigester) ergibt 11-Chlor-1,2,4,5,6,7-hexahdydro-4-benzazecin-3,8-dion, als beige Kristalle vom Schmelzpunkt 142-144°.

Beispiel 11

Man versetzt eine Lösung von 2,05 g (7 mMol) 4-Acetyl-11-chlor-1,2,4,5,6,7-hexahydro-4-benzazecin-3,8-dion in 30 ml Methanol mit 0,85 g (15,4 mMol) Natriummethanolat, rührt 2 Stunden bei Raumtemperatur, engt das Reaktionsgemisch im Vakuum ein, versetzt mit Wasser und extrahiert bei pH 14 mit Chloroform. Nach Trocknen der Chloroformphase mit Natriumsulfat und Abdestillieren des Lösungsmittels im Vakuum erhält man 11-Chlor-1,2,4,5,6,7-hexahydro-4-benzazecin-3,8-dionvom Schmelzpunkt 142-144°.

Beispiel 12

a) Man löst 15,0 g (0,06 Mol) 8-Chlor-1,2,3,4,5,6-hexahydrobenzo[f]chinolin in 150 ml Methylenchlorid, versetzt mit 6,68 g (0,066 Mol) Triäthylamin und tropft bei 0° 11,2 g (0,066 Mol) p-Methoxybenzoylchlorid in 50 ml Methylenchlorid zu. Nach 1-stündigem Rühren bei Raumtemperatur extrahiert man mit Wasser, trocknet mit Magnesiumsulfat und destilliert das Lösungsmittel im Vakuum ab. Kristallisieren (Methylenchlorid-Aether) ergibt 4-(p-Methoxybenzoyl)-8-chlor-1,2,3,4,5,6 -hexahydrobenzo[f]chinolin als weisse Kristalle vom Schmelzpunkt 182-183°.

b) Man löst 8,80 g (0,025 Mol) 4-(p-Methoxybenzoyl)-8-chlor-1,2,3,4,5,6 -hexahydrobenzo[f]chinolin in 100 ml Chloroform und tropft bei 0° ein Suspension von 10,3 g (0,051 Mol) 85%ige m-Chlorperbenzoesäure in 100 ml Chloroform zu. Nach 1-stündigem Rühren bei Raumtemperatur extrahiert man mit 2N Natronlauge und Wasser trocknet mit Magnesiumsulfat und destilliert das Lösungsmittel im Vakuum ab. Chromatographieren (Kieselgel, Aether-Hexan, 2:1) und Kristallisieren (Aether-Hexan) ergibt 4-(p-Methoxybenzoyl)-11-chlor-1,2,4,5,6,7-hexahydro -4-benzazecin-3,8-dion als weisse Kristalle vom Schmelzpunkt 143°.

Beispiel 13

a) Man löst 8,78 g (40 mMol) 8-Chlor-1,2,3,4,5,6-hexahydrobenzo[f]chinolinin 88 ml Methylenchlorid und tropft bei 0° 7,70 g (44 mMol) 4-Chlorbenzoylchlorid in 60 ml Methylenchlorid zu. Nach 1-stündigem Rühren bei Raumtemperatur extrahiert man mit Wasser, trocknet mit Magnesiumsulfat und destilliert das Lösungsmittel im Vakuum ab. Chromatographieren (Kieselgel, Chloroform-Hexan 2:1) und Kristallisieren (Chloroform-Hexan) ergibt 8-Chlor-4-(p-chlorbenzoyl)-1,2,3,4,5,6 -hexahydrobenzo[f]chinolin als weisse Kristalle vom Schmelzpunkt 189-191°.

b) Man löst 5,20 g (14,5 mMol) 8-Chlor-4-(p-chlorbenzoyl)-1,2,3,4,5,6 -hexahydrobenzo[f]chinolin in 50 ml Chloroform und tropft bei 0° eine Suspension von 5,90 g (29 mMol) 85%ige m-Chlorperbenzoesäure in 50 ml Chloroform zu. Nach 1-stündigem Rühren bei Raumtemperatur extrahiert man mit 2N Natronlauge und Wasser, trocknet mit Magnesiumsulfat und destilliert das Lösungsmittel im Vakuum ab. Chromatographieren (Kieselgel, Chloroform) und Kristallisieren (Aether-Hexan) ergibt 11-Chlor-4-(p-chlorbenzoyl)-1,2,4,5,6,7-hexahydro -4-benzazecin-3,8-dion als weisse Kristalle vom Schmelzpunkt 141-143°.

Beispiel 14

a) Man löst 14,3 g (0,066 Mol) 8-Chlor-1,2,3,4,5,6-hexahydrobenzo[f]chinolin in 143 ml Methylenchlorid, versetzt mit 6,68 g (0,066 Mol) Triäthylamin und tropft bei 0° 11,3 g (0,066 Mol) m-Methoxybenzoylchlorid in 60 ml Methylenchlorid zu. Nach 1-stündigem Rühren bei Raumtemperatur extrahiert man mit Wasser, trocknet mit Magnesiumsulfat und destilliert das Lösungsmittel im Vakuum ab. Chromatographieren (Kieselgel, Toluol-Essigester, 19:1) und Kristallisieren (Methylenchlorid-Aether) ergibt 4-(m-Methoxybenzoyl)-8-chlor-1,2,3,4,5,6 -hexahydrobenzo[f]chinolin als weisse Kristalle vom Schmelzpunkt 133°.

b) Man löst 3,20 g (0,009 Mol) 4-(m-Methoxybenzoyl)-8-chlor-1,2,3,4,5,6 -hexahydrobenzo[f]chinolin in 35 ml Methylenchlorid und tropft bei 0° eine Suspension von 3,77 g (0,019 Mol) 85-proz. m-Chlorperbenzoesäure in 35 ml Methylenchlorid zu. Nach 1-stündigem Rühren bei Raumtemperatur extrahiert man mit 2N Natronlauge und Wasser, trocknet mit Magnesiumsulfat und destilliert das Lösungsmittel im Vakuum ab. Kristallisieren (Methylenchlorid-Aether) ergibt 4-(m-Methoxybenzoyl)-11-chlor-1,2,4,5,6,7-hexahydro -4-benzazecin-3,8-dion als weisse Kristalle vom Schmelzpunkt 105-106°.

Beispiel 15

a) Man löst 5,00 g (0,02 Mol) 8-Chlor-1,2,3,4,5,6-hexahydro[f]chinolin in 70 ml Methylenchlorid, versetzt mit 2,23 g (0,022 Mol) Triäthylamin und tropft bei 0° 3,85 g (0,022 Mol) o-Methoxybenzoylchlorid in 20 ml Methylenchlorid zu. Nach 1-stündigem Rühren bei Raumtemperatur extrahiert man mit Wasser, trocknet mit Magnesiumsulfat und destilliert das Lösungsmittel im Vakuum ab. Chromatographieren (Kieselgel. Toluol-Essigester, 9:1) und Kristallisieren (Methylenchlorid-Aether) ergibt 4-(o-Methoxybenzoyl)-8-chlor-1,2,3,4,5,6 -hexahydrobenzo[f]chinolin als weisse Kristalle vom Schmelzpunkt 139°.

b) Man löst 5,75 g (0,016 Mol) 4-(o-Methoxybenzoyl)-8-chlor-1,2,3,4,5,6 -hexahydrobenzo[f]chinolin in 60 ml Methylenchlorid und tropft bei 0° eine Suspension von 6,70 g (0,033 Mol) 85-proz. m-Chlorperbenzoesäure in 60 ml Methylenchlorid zu. Nach 1-stündigem Rühren bei Raumtemperatur extrahiert man mit 2N Natronlauge und Wasser, trocknet mit Magnesiumsulfat und destilliert das Lösungsmittel im Vakuum ab. Kristallisieren (Methylenchlorid-Aether) ergibt 4-(o-Methoxybenzoyl)-11-chlor-1,2,4,5,6,7-hexahydro

-4-benzazecin-3,8-dion als weisse Kristalle vom Schmelzpunkt 179°.

Beispiel 16

a) Zu 110,7 g Aluminiumchlorid in 800 ml Methylenchlorid werden unter Rühren zwischen 0° und 5° innerhalb von 50 Minuten 96,9 g p-Bromphenylessigsäurechlorid, gelöst in 200 ml Methylenchlorid, zugetropft. Danach wird in das Gemisch zwischen 0-5° während 30 Minuten Aethylen eingeleitet, worauf man noch 1 Stunde bei Raumtemperatur rührt und dann zwischen 0° und 3° 600 ml kaltes Wasser zugibt. Die Methylenchloridphase wird abgetrennt und mit 2 × 350 ml 2N Salzsäure und 2 × 350 ml gesättigter Natriumbicarbonatlösung gewaschen. Die Wasserphasen werden mit 200 ml Methylenchlorid extrahiert. Die vereinigten Methylenchloridextrakte werden über Natriumsulfat getrocknet und im Vakuum eingeengt. Das aus 400 ml tiefsiedendem Petroläther auskristallisierte 6-Brom-3,4-dihydro-2(1H)-naphthalinon zeigt einen Schmelzpunkt von 75-77°.

b) 35,0 g 6-Brom-3,4-dihydro-2(1H)-naphthalinon werden in 250 ml Benzol und 12,5 ml Pyrrolidin in Gegenwart von 0,5 g p-Toluolsulfonsäure 3 Stunden am Rückfluss gekocht. Nach Abdampfen des Toluols im Vakuum wird der Rückstand in Diäthyläther verrührt. Das auskristallisierte 1-(6-Brom-3,4-dihydro-2-naphthyl)pyrrolidin zeigt einen Schmelzpunkt von 125-127°. Aus der Mutterlauge kann durch Verrühren in Isopropyläther eine weitere Portion des obigen Produkts vom Schmelzpunkt 127-128° isoliert werden.

c) Zu 35,5 g 1-(6-Brom-3,4-dihydro-2-naphthyl)pyrrolidin werden 17,5 g Acrylamid und 0,65 g p-Toluolsulfonsäure zugegeben. Man erhitzt unter Stickstoff je 2 Stunden auf 100° und auf 150°. Das Reaktionsgemisch wird mit 250 ml Chloroform versetzt, worauf man die unlöslichen Anteile abfiltriert. Man erhält 8-Brom-1,4,5,6-tetrahydrobenzo[f]chinolin-3(2H)-on vom Schmelzpunkt 227-228°. Nach Umkristallisieren aus Aethanol zeigt das Produkt einen Schmelzpunkt von 230-231°.

d) Zu einer bei 20° unter Stickstoff gerührten Suspension von 5,2 g Lithiumaluminiumhydrid in 140 ml trockenem Tetrahydrofuran werden innerhalb von 35 Minuten zwischen 20° und 25° portionenweise 19,0 g 8-Brom-1,4,5,6-tetrahydrobenzo[f]-chinolin-3(2H)-on zugegeben. Das Reaktionsgemisch wird anschliessend während 150 Minuten unter Rückfluss zum Sieden erhitzt, dann abgekühlt und hierauf zwischen 0° und 10° mit 17 ml 6,5N Natronlauge versetzt. Die entstandene Suspension wird filtriert, worauf man den Filterrückstand mehrmals mit Tetrahydrofuran nachwäscht und das Filtrat im Vakuum eindampft. Das so erhaltene 8-Brom-1,2,3,4,5,6-hexahydrobenzo[f]chinolin wird direkt weiterverarbeitet.

e) 13,9 g rohes 8-Brom-1,2,3,4,5,6-hexahydrobenzo[f]chinolin werden in 29 ml Pyridin und 27,0 ml Essigsäureanhydrid aufgenommen. Man lässt das Reaktionsgemisch 20 Stunden bei Raumtemperatur stehen, dampft ein, nimmt den verbleibenden Rückstand zweimal in je 100 ml trockenem Toluol auf und dampft die erhaltenen Lösungen zur Trockene ein. Der Rückstand wird in Essigester gelöst und an 200 g Kieselgel (Korngrösse 0,2-0,5 mm) chromatographiert. Das mit Essigester eluierte 4-Acetyl-8-brom-1,2,3,4,5,6-hexahydrobenzo[f]chinolin zeigt nach Umkristallisieren aus Isopropyläther einen Schmelzpunkt von 96-97°.

f) 8,0 g 4-Acetyl-8-brom-1,2,3,4,5,6-hexahydrobenzo[f]chinolin werden in 80 ml Chloroform gelöst, worauf zwischen 0° und +5° 11,2 g 85%ige m-Chlorperbenzoesäure, gelöst in 80 ml Chloroform, tropfenweise zugegeben werden. Danach wird bei Raumtemperatur während 4 Stunden nachgerührt, worauf man 1,0 g Kaliumjodid und 200 ml Wasser zugibt und dann bis zur Entfärbung mit Natriumthiosulfat versetzt. Die Chloroformphase wird abgetrennt und mit 100 ml 2N Natronlauge und mit 200 ml Wasser gewaschen. Die Wasserphasen werden mit 100 ml Chloroform extrahiert. Die vereinigten Chloroformextrakte werden getrocknet und im Vakuum eingedampft. Das aus Essigester umkristallisierte 4-Acetyl-11-brom-1,2,4,5,6,7-hexahydro-4-benzazecin-3,8-dion zeigt einen Schmelzpunkt von 162-164°.

Beispiel 17

a) 12,0 g 6-Methoxy-2-tetralon werden in 200 ml Benzol und 5,6 ml Pyrrolidin in Gegenwart von 0,5 g wasserfreier p-Toluolsulfonsäure 2 Stunden am Rückfluss gekocht. Das Toluol wird im Vakuum entfernt. Zum so erhaltenen rohen 1-(6-Methoxy-3,4-dihydro-2-naphthyl)pyrrolidin werden 9,70 g Acrylamid und 0,5 g p-Toluolsulfonsäure zugegeben. Man erhitzt unter Stickstoff je 2 Stunden auf 100° und auf 150°. Das Reaktionsgemisch wird zwischen 400 ml Chloroform und 40 ml Wasser verteilt. Die organische Phase wird mit 2 × 40 ml Wasser gewaschen, und die Wasserphasen werden mit 1 × 100 ml Chloroform extrahiert. Die Chloroformphasen werden verenigt) über Natriumsulfat getrocknet und eingeengt. Der Rückstand wird an 160 g Kieselgel (Korngrösse 0,2-0,5 mm) chromatographiert. das mit Methylenchlorid eluierte und aus Essigester umkristallisierte 8-Methoxy-1,4,5,8-tetrahydrobenzo[f]-chinolin-3(2H)-on zeigt einen Schmelzpunkt von 208-209°.

b) Zu einer bei 20° unter Stickstoff gerührten Suspension von 1,69 g Lithiumaluminiumhydrid in 50 ml trockenem Tetrahydrofuran werden innerhalb von 35 Minuten zwischen 20° und 25° portionenweise 5,1 g 8-(Methoxy-1,4,5,8-tetrahydrobenzo[f]chinolin-3(2H)-on zugegeben. Das Reaktionsgemisch wird anschliessend während 150 Minuten unter Rückfluss zum Sieden erhitzt, dan abgekühlt und hierauf zwischen 0° und 10° mit 1,5 ml 6,5N Natronlauge versetzt. Die entstandene Suspension wird filtriert, worauf man den Filterrückstand mehrmals mit Tetrahydrofuran nachwäscht und das Filtrat im Vakuum eindampft. Das so erhaltene 8-Methoxy-1,2,3,4,5,6-hexahydrobenzo[f]chinolin wird direkt weiterverarbeitet.

c) 4,7 g 8-Methoxy-1,2,3,4,5,6-hexahydrobenzo[f]chinolin werden in 15 ml Pyridin und 11 ml Essigsäureanhydrid aufgenommen. Man lässt das Reaktionsgemisch 20 Stunden bei Raumtemperatur stehen, dampft dann ein, nimmt den verbleibenden Rückstand zweimal in je 50 ml trockenem Toluol auf und dampft die erhaltenen Lösungen zur Trockene ein. Der Rückstand wird in Methylenchlorid gelöst und an 50 g Kieselgel (Korngrösse 0,2-0,5 mm) chromatographiert. Das mit Methylenchlorid eluierte 4-Acetyl-8-methoxy-1,2,3,4,5,6-hexahydrobenzo[f]chinolin zeigt nach Umkristallisieren aus Isopropyläther einen Schmelzpunkt von 119-120°.

d) 21,3 g 4-Acetyl-8-methoxy-1,2,3,4,5,6-hexahydrobenzo[f]chinolin werden in 200 ml Chloroform gelöst, worauf bei 0° bis +5° 38,0 g 85%ige m-Chlorperbenzoesäure, gelöst in 250 ml Chloroform, tropfenweise zugegeben werden. Danach wird bei Raumtemperatur 18 Stunden gerührt, worauf man Natriumjodid und Wasser zugibt und danach bis zur Entfärbung mit Natriumthiosulfat versetzt. Die Chloroformphase wird mit wässerigem Ammoniak und Natriumchloridlösung gewaschen. Die Wasserphasen werden mit Chloroform extrahiert. Die vereinigten Chloroformextrakte werden über Natriumsulfat getrocknet und im Vakuum eingedampft. Das aus Essigester umkristallisierte 4-Acetyl-11-methoxy-1,2,4,5,6,7-hexahydro-4-benzazecin -3,8-dion zeigt einen Schmelzpunkt von 156-158°.

Beispiel 18

a) 20,6 g 5-Chlor-1-tetralon werden in 100 ml Alkohol gelöst, worauf portionenweise 2,2 g Natriumborhydrid zugegeben werden. Das Reaktionsgemisch wird 2 Stunden bei 40° gerührt, dann abgekühlt und hierauf bei Raumtemperatur mit 32 ml 2N Salzsäure versetzt. Dann engt man im Vakuum ein und extrahiert mit Methylenchlorid. Die organischen Phasen werden über Natriumsulfat getrocknet und eingedampft. Das als Rückstand verbleibende 5-Chlor-1-tetralol wird als Rohprodukt direkt weiterverarbeitet.

b) 20,6 g rohes 5-Chlor-1-tetralol werden in 400 ml Toluol gelöst, und zu dieser Lösung werden 1,60 g p-Toluolsulfonsäure zugegeben. Das Gemisch wird für 2 Stunden am Rückfluss erhitzt, wobei mit einem Wasserabscheider 1,9 ml Wasser aufgefangen werden. Hierauf gibt man 3 g Molekularsieb 4A zu und erhitzt weitere 30 Minuten am Rückfluss. Das Reaktionsgemisch wird abgekühlt; die organische Phase wird einmal mit 100 ml gesättigter wässeriger Natriumbicarbonatlösung und zweimal mit je 50 ml Wasser gewaschen, getrocknet und im Vakuum eingedampft. Das als Rückstand verbleibende 5-Chlor-3,4-dihydronaphthalin wird im Kugelrohr destilliert; Siedepunkt 100°/0,05mmHg.

c) Zu 23,2 g m-Chlorperbenzoesäure (85%ig), gelöst in 320 ml Methylenchlorid, werden zwischen 0° und +5° innerhalb von 20 Minuten tropfenweise 17,0 g 5-Chlor-3,4-dihydronaphthalin, gelöst in 300 ml Methylenchlorid, zugegeben. Das Gemisch wird anschliessend 3,5 Stunden bei Raumtemperatur gerührt und dann sechsmal mit je 100 ml 5%igem wässerigem Ammoniak gewaschen. Die Methylenchloridphase wird über Natriumsulfat getrocknet, filtriert und im Vakuum eingedampft. Das als Rückstand verbleibende 5-Chlor-oxirano[α]-2,3-dihydronaphthalin wird als Rohprodukt weiterverarbeitet.

d) 24 g rohes 5-Chloroxirano[α]-2,3-dihydronaphthalin werden in 350 ml Toluol gelöst. Zu dieser Lösung gibt man unter Stickstoff bei Raumtemperatur tropfenweise eine frisch zubereitete Magnesiumbromidlösung [4,25 g Magnesiumspäne werden in 800 ml Diäthyläther unter Stickstoff mit 8,2 ml (25,5 g) Brom versetzt, worauf 30 Minuten gekocht und von den unlöslichen Anteilen abdekantiert wird]. Anschliessend wird 1 Stunde bei Raumtemperatur gerührt. Der Diäthyläther wird im Vakuum abdestilliert und kontinuierlich durch 1000 ml Toluol ersetzt. Das erhaltene Gemisch wird 2 Stunden unter Rückfluss gekocht, auf Raumtemperatur abgekühlt und dann mit 300 ml und 2 x je 100 ml kaltem Wasser gewaschen. Die wässerigen Phasen werden mit 150 ml Toluol nachextrahiert. Die vereinigten organischen Phasen werden über Natriumsulfat getrocknet und eingeengt. Der Rückstand wird über 100 g Kieselgel (Korngrösse 0,04-0,063 mm) chromatographiert. Das mit Toluol eluierte 5-Chlor-2-tetralon wird zur weiteren Reinigung im Kugelrohr destilliert (Siedepunkt 145-150°/0,04mmHg): das kristallisierte Produkt zeigt einen Schmelzpunkt von 32-34° (Sintern ab 31°).

e) 11,07 g 5-Chlor-2-tetralon werden in 200 ml Benzol und 10,1 ml Pyrrolidin in Gegenwart von 0,3 g p-Toluolsulfonsäure 2 Stunden am Rückfluss gekocht. Das erhaltene rohe 1-(5-Chlor-3,4-dihydro-2-naphthyl)pyrrolidin wird ohne Reinigung mit 9,0 g Acrylamid und 0,5 g p-Toluolsulfonsäure versetzt. Man erhitzt unter Stickstoff je 2 Stunden auf 100° und auf 150°. Zum Reaktionsgemisch werden 250 ml Essigsäureäthylester und 50 ml Wasser zugegeben, worauf man die unlöslichen Anteile abfiltriert, mit Essigsäureäthylester und Wasser wäscht, trocknet und aus Esssigsäureäthylester umkristallisiert. Man erhält 7-Chlor-1,2,5,6-tetrahydrobenzo[f]chinolin-3(4H)-on vom Schmelzpunkt 252-253°.

f) Zu einer unter Stickstoff gerührten Suspension von 1,30 g Lithiumaluminiumhydrid in 60 ml trockenem Tetrahydrofuran werden innerhalb von 35 Minuten zwischen 20° und 25° portionenweise 4,0 g 7-Chlor-1,2,5,6-tetrahydrobenzo[f]chinolin-3(4H)-on zugegeben. Das Reaktionsgemisch wird anschliessend während 150 Minuten unter Rückfluss zum Sieden erhitzt, dann abgekühlt und hierauf zwischen 0° und 10° mit 4,2 ml 6,5N Natronlauge versetzt. Die entstandene Suspension wird filtriert, worauf man den Filterrückstand mit 5 x je 20 ml Tetrahydrofuran nachwäscht und das Filtrat im Vakuum eindampft. Das so erhaltene rohe 7-Chlor-1,2,3,4,5,6-hexahydrobenzo[f]chinolin wird in 10 ml Pyridin und 9,0 ml Essigsäureanhydrid aufgenommen. Man lässt das Reaktionsgemisch 20 Stunden bei Raumtemperatur stehen, dampft ein, nimmt den verbleibenden Rückstand zweimal in je 50 ml trockenem Toluol auf und dampft die erhaltenen Lösungen zur Trockene ein. Der Rückstand wird in Methylenchlorid gelöst und an 70 g Kieselgel (Korngrösse 0,04-0,063 mm) chromatographiert. Das mit Chloroform eluierte 4-Acetyl-7-chlor-1,2,3,4,5,6-hexahydrobenzo[f]chinolin zeigt nach Umkristallisieren aus Isopropyläther einen Schmelzpunkt von 89-90°.

g) 3,4 g 4-Acetyl-7-chlor-1,2,3,4,5,6-hexahydrobenzo[f]chinolin werden in 100 ml Chloroform gelöst, worauf bei 0° bis +5° 6,8 g 85%ige m-Chlorperbenzoesäure, gelöst in 100 ml Chloroform, tropfenweise zugegeben werden. Nach 3-stündigem Rühren bei Raumtemperatur werden 1,5 g Kaliumjodid und 40 ml Wasser zugegeben, worauf man bis zur Entfärbung mit Natriumthiosulfat versetzt. Die Chloroformphase wird mit 40 ml 2N Natronlauge und 2 x 80 ml Wasser gewaschen. Die Wasserphasen werden mit 100 ml Chloroform ausgeschüttelt. Die vereinigten Chloroformextrakte werden getrocknet und im Vakuum eingedampft. Das aus Essigsäureäthylester umkristallisierte 4-Acetyl-12-chlor-1,2,4,5,6,7-hexahydro-4-benzazecin-3,8-dion zeigt einen Schmelzpunkt von 160-162°.

Beispiel 19

a) In 1125 ml Aethanol werden 75,0 g 5-Methoxy-1-tetralon gelöst, worauf bei Raumtemperatur portionenweise 8,05 g Natriumborhydrid zugegeben werden. Das Reaktionsgemisch wird 3,5 Stunden auf 40° erwärmt, dann auf Raumtemperatur abgekühlt und hierauf bei 20-26° mit 120 ml 2N Salzsäure versetzt. Dann engt man im Vakuum ein und extrahiert mit Methylenchlorid. Die organischen Phasen werden über Natriumsulfat getrocknet und eingedampft. Das als Rückstand verbleibende 1,2,3,4-Tetrahydro-5-methoxy-1-naphthalinol zeigt nach Umkristallisieren aus n-Hexan einen Schmelzpunkt von 78,5-79°.

b) 51,0 g 1,2,3,4-Tetrahydro-5-methoxy-1-naphthalinol werden in 1.7 l Toluol mit 4,0 g p-Toluolsulfonsäure 2 Stunden am Wasserabscheider gekocht. Das Reaktionsgemisch wird mit 200 ml gesättigter Natriumbicarbonatlösung und mit 2 x 200 ml Wasser gewaschen. Die Toluolphase wird über Natriumsulfat getrocknet und im Vakuum eingedampft, wobei roher 7,8-Dihydro-1-naphthylmethyläther als Rückstand verbleibt. Zur Reinigung wird das erhaltene Rohprodukt über 600 g Kieselgel (Korngrösse 0,2-0,5 mm) chromatographiert. Der mit n-Hexan eluierte 7,8-Dihydro-1-naphthylmethyläther wird ohne weitere Reinigung direkt weiterverarbeitet. Eine Probe wird im Kugelrohr destilliert und zeigt dabei einen Siedepunkt von 110°/0,05mmHg.

c) Zu 50,3 g 85%iger m-Chlorperbenzoesäure in 800 ml Methylenchlorid werden zwischen 0° und +5° 36 g 7,8-Dihydro-1-naphthylmethyläther, gelöst in 600 ml Methylenchlorid, zugetropft. Anschliessend wird 5,5 Stunden bei Raumtemperatur gerührt und dann mit 6 x 200 ml 5%igem wässerigen Ammoniak gewaschen. Die Wasserphasen werden mit 300 ml Methylenchlorid extrahiert. Die organischen Phasen werden vereinigt, über Natriumsulfat getrocknet und eingeengt. Das so erhaltene 5-Methoxyoxirano[α]-2,3-dihydronaphthalin wird ohne vorangehende Reinigung in 800 ml Toluol gelöst. Zu dieser Lösung gibt man tropfenweise unter Stickstoff bei Raumtemperatur eine frisch zubereitete Magnesiumbromidlösung [9,2 g Magnesiumspäne werden in 1,7 l Diäthyläther unter Stickstoff mit 17,8 ml (55,2 g) Brom versetzt, worauf 30 Minuten gekocht und von den unlöslichen Anteilen abdekantiert wird]. Anschliessend wird 1 Stunde bei Raumtemperatur gerührt. Der Diäthyläther wird im Vakuum abdestilliert und kontinuierlich durch 2 l Toluol ersetzt. Die erhaltene Lösung wird 3 Stunden unter Rückfluss gekocht, dann auf Raumtemperatur abgekühlt und mit 3 x 200 ml kaltem Wasser gewaschen, worauf die Wasserphasen mit 300 ml Toluol extrahiert werden. Die organischen Phasen werden vereinigt, über Natriumsulfat getrocknet und eingeengt. Das verbleibende rohe 3,4-Dihydro-5-methoxy-2-(1H)-naphthalinon wird über

500 g Kieselgel (Korngrösse 0,2-0,5 mm) chromatographiert. Das mit Toluol eluierte Produkt wird zur weiteren Reinigung im Kugelrohr destilliert und zeigt dann einen Schmelzpunkt von 37,5-38,5°.

d) 10,18 g 3,4-Dihydro-5-methoxy-2(1H)-naphthalinon werden in 200 ml Benzol und 4,8 ml Pyrrolidin in Gegenwart von 0,25 g p-Toluolsulfonsäure 2 Stunden am Rückfluss gekocht. Das Toluol wird im Vakuum abdestilliert, und der Rückstand wird in tiefsiedendem Petroläther verrührt. Das 1-(5-Methoxy-3,4-dihydro-2-naphthyl)pyrrolidin wird abfiltriert und zeigt einen Schmelzpunkt von 77-78°.

e) Zu 9,9 g 1-(5-Methoxy-3,4-dihydro-2-naphthyl)pyrrolidin werden 7,3 g Acrylamid und 0,4 g p-Toluolsulfonsäure zugegeben. Man erhitzt das Gemisch unter Stickstoff je 2 Stunden auf 100° und auf 150° und gibt dann 150 ml Chloroform und 30 ml Wasser zu. Das ausgefallene 1,4,5,6-Tetrahydro-7-methoxy-3(2H)-chinolinon wird abfiltriert und zeigt einen Schmelzpunkt von 248-250°.

f) Zu einer bei 20° unter Stickstoff gerührten Suspension von 3,1 g Lithiumaluminiumhydrid in 120 ml trockenem Tetrahydrofuran werden innerhalb von 35 Minuten zwischen 20° und 25° portionenweise 9,1 g 1,4,5,6-Tetrahydro-7-methoxy-3(2H)-chinolinon zugegeben. Das Reaktionsgemisch wird anschliessend während 150 Minuten unter Rückfluss zum Sieden erhitzt, dann abgekühlt und hierauf zwischen 0° und 10° mit 9,7 ml 6,5N Natronlauge versetzt. Die entstandene Suspension wird filtriert, worauf man den Filterrückstand mehrmals mit je 20 ml Tetrahydrofuran nachwäscht und die vereinigten Filtrate in Vakuum eindampft. Das so erhaltene 1,2,3,4,5,6-Hexahydro-7-methoxybenzo[f]chinolin wird direkt weiterverarbeitet.

g) 9,0 g 1,2,3,4,5,6-Hexahydro-7-methoxybenzo[f]chinolin werden in 20 ml Pyridin und 17,5 ml Essigsäureanhydrid aufgenommen. Man lässt das Reaktionsgemisch 20 Stunden bei Raumtemperatur stehen, dampft dann ein, nimmt den verbleibenden Rückstand zweimal in je 75 ml trockenem Toluol auf und dampft die erhaltenen Lösungen zur Trockene ein. Der Rückstand wird in Essigester gelöst und an 100 kg Kieselgel (Korngrösse 0,2-0,5 mm) chromatographiert. Das mit Essigester eluierte und aus Isopropyläther umkristallisierte 4-Acetyl-1,2,3,4,5,6-hexahydro-7-methoxybenzo[f]chinolin zeigt einen Schmelzpunkt von 128-130°.

h) 4,87 g 4-Acetyl-1,2,3,4,5,6-hexahydro-7-methoxybenzo[f]chinolin werden in 120 ml Chloroform gelöst, worauf bei 0° bis +5° 9,55 g 85%ige m-Chlorperbenzoesäure, gelöst in 120 ml Chloroform, tropfenweise zugegeben werden. Danach wird bei Raumtemperatur 3 Stunden gerührt, worauf man 1,6 g Kaliumjodid und 40 ml Wasser zugibt und dann bis zur Entfärbung mit Natriumthiosulfat versetzt. Die Chloroformphase wird mit 40 ml 2N Natronlauge und 2 × 100 ml Wasser gewaschen. Die Wasserphasen werden mit 100 ml Chloroform extrahiert. Die vereinigten Chloroformextrakte werden getrocknet und im Vakuum eingedampft. Der Rückstand wird an 60 g Kieselgel (Korngrösse 0,2-0,5 mm) chromatographiert. Das mit Methylenchlorid eluierte und aus Essigester umkristallisierte 4-Acetyl-1,2,4,5,6,7-hexahydro-12-methoxy -4-benzazecin-3,8-dion zeigt einen Schmelzpunkt von 170-172°.

Beispiel 20

a) Zu 115 g Aluminiumchlorid in 700 ml Methylenchlorid werden unter Rühren bei 0° bis 5° innerhalb von 60 Minuten 149,4 g 2,4-Dichlorphenylacetylchlorid, gelöst in 200 ml Methylenchlorid, zugetropft. Danach wird zwischen 0° und 5° während 30 Minuten Aethylen eingeleitet, worauf man 1 Stunde bei Raumtemperatur weiterrührt und danach zwischen 0° und 5° mit 300 ml Eiswasser versetzt. Die Methylenchloridphase wird mit 2N Salzsäure, mit Wasser, mit gesättiger Natriumbicarbonatlösung und nochmals mit Wasser gewaschen. Die wässerigen Phasen werden mit Methylenchlorid extrahiert. Die Methylenchloridphasen werden vereinigt, getrocknet und eingedampft. Man verrührt den Rückstand mit 100 ml tiefsiedendem Petroläther, gibt später weitere 250 ml tiefsiedenden Petroläther zu und lässt 72 Stunden im Eisschrank stehen. Der Festkörper wird abfiltriert und unter Eluieren mit Chloroform über 600 g Kieselgel (Korngrösse 0,2-0,5 mm) filtriert. Man erhält 6,8-Dichlor-3,4-dihydro-2(1H)-naphthalinon, das nach Umkristallisieren aus tiefsiedendem Petroläther bei 0° bis 5° einen Schmelzpunkt von 90-92° zeigt.

b) 30,0 g 6,8-Dichlor-3,4-dihydro-2(1H)-naphthalinon werden in 250 ml Benzol und 11.5 ml Pyrrolidin in Gegenwart von 0,5 g wasserfreiem p-Toluolsulfonsäure 2 Stunden am Rückfluss gekocht. Das erhaltene 1-(6,8-Dichlor-3,4-dihydro-2-naphthyl)pyrrolidinwird zur Reinigung mit 150 ml Isopropyläther verrührt. Der abfiltrierte Festkörper zeigt einen Schmelzpunkt von 84-86°. Aus den Mutterlaugen kann eine weitere Portion vom Schmelzpunkt 84,5-85,5° isoliert werden. Die beiden Portionen werden vereinigt, und nach Umkristallisieren aus Isopropyläther erhält man 1-(6,8-Dichlor-3,4-dihydro-2-naphthyl)pyrrolidin vom Schmelzpunkt 85,5-86°.

c) Zu 30.6 g 1-(6,8-Dichlor-3,4-dihydro-2(1H)-naphthyl)-pyrrolidin werden 16.2 g Acrylamid und 0,9 g wasserfreie p-Toluolsulfonsäure zugegeben. Man erhitzt unter Stickstoff je 2 Stunden auf 100° und auf

150°. Nach dem Abkühlen wird das Reaktionsgemisch bei Raumtemperatur in 80 ml eines Gemisches von Chloroform-Essigester (1:1) verrührt. Man erhält 8,10-Dichlor-1,4,5,6-tetrahydrobenzo[f]chinolin-3-(2H)-on, welches nach Umkristallisieren aus Essigester bei 212-214° schmilzt.

d) Zu einer bei 20° unter Stickstoff gerührten Suspension von 2,26 g Lithiumaluminiumhydrid in 67 ml trockenem Tetrahydrofuran werden innerhalb von 35 Minuten zwischen 20-25° portionenweise 8,0 g 8,10-Dichlor-1,4,5,6-tetrahydrobenzo-[f]chinolin-3(2H)-on zugegeben. Das Reaktionsgemisch wird anschliessend während 150 Minuten unter Rückfluss zum Sieden erhitzt, danach abgekühlt und zwischen 0° und +10° mit 7,38 ml 6,5N Natronlauge versetzt. Die entstandene Suspension wird filtriert, und das Filtrat wird im Vakuum eingedampft. Man nimmt das so erhaltene 8,10-Dichlor-1,2,3,4,5,6-hexahydrobenzo[f]chinolin in 16 ml Pyridin und 14 ml Essigsäureanhydrid auf, lässt über Nacht bei Raumtemperatur stehen, dampft ein, nimmt den verbleibenden Rückstand zweimal in je 50 ml Toluol auf und dampft die erhaltenen Lösungen zur Trockene ein. Der Rückstand wird in Chloroform gelöst und an 100 g Kieselgel (Korngrösse 0,2-0,5 mm) chromatographiert. Das mit Chloroform eluierte 4-Acetyl-8,10-dichlor-1,2,3,4,5,6-tetrahydrobenzo[f]chinolin zeigt nach Umkristallisieren aus Isopropyläther einen Schmelzpunkt von 99-101°.

e) 4,2 g 4-Acetyl-8,10-dichlor-1,2,3,4,5,6-tetrahydrobenzo-[f] chinolin werden in 80 ml Chloroform gelöst, worauf bei 0° bis +5° 7,45 g 85%ige m-Chlorperbenzoesäure, gelöst in 80 ml Chloroform, tropfenweise Zugegeben werden. Danach wird bei Raumtemperatur 3 Stunden gerührt, worauf man 1,5 g Kaliumjodid und 50 ml Wasser zugibt und dann bis zur Entfärbung mit Natriumthiosulfat versetzt. Die Chloroformphase wird mit 40 ml 2N Natronlauge und 2 x 100 ml Wasser gewaschen. Die Wasserphasen werden mit 100 ml Chloroform extrahiert. Die vereinigten Chloroformextrakte werden getrocknet und im Vakuum eingedampft. Das aus Isopropyläther umkristallisierte 4-Acetyl-9,11-dichlor-1,2,4,5,6,7-hexahydro -4-benzazecin-3,8-dion zeigt einen Schmelzpunkt von 152-153°.

Beispiel A

Man stellt Tabletten folgender Zusammensetzung her, welche als Wirkstoff 4-Acetyl-11-chlor-1,2,4,5,6,7-hexahydro -4-benzazecin-3,8-dion enthalten.

| | | pro Tablette |
|---|---|---|
| 1. | Wirkstoff (mikronisiert) | 50 mg |
| 2. | Milchzucker | 120 mg |
| 3. | Maisstärke | 50 mg |
| 4. | Polyvinylpyrrolidon | 8 mg |
| 5. | Natrium-carboxymethylstärke | 20 mg |
| 6. | Magnesiumstearat | 2 mg |
| | | 250 mg |

Der Wirkstoff wird mit einer Mischung aus Milchzucker und Maisstärke homogen vermischt. Man siebt, befeuchtet mit einer wässerigen Polyvinylpyrrolidonlösung, granuliert und trocknet. Das getrocknete Granulat wird mit Natrium-carboxymethylstärke und Magnesiumstearat vermischt, und die so erhaltene Mischung wird zu Tabletten geeigneter Grösse mit Bruchrille verpresst.

Beispiel B

Man stellt Tabletten folgender Zusammensetzung her, welche als Wirkstoff 4-Acetyl-11-chlor-1,2,4,5,6,7-hexahydro-4-benzazecin-3,8-dion enthalten.

| | | pro Tablette |
|---|---|---|
| 1. | Wirkstoff (mikronisiert) | 10 mg |
| 2. | Milchzucker | 88 mg |
| 3. | Mikrokristalline Cellulose | 60 mg |
| 4. | Maisstärke | 20 mg |
| 5. | Natrium-carboxymethylstärke | 20 mg |
| 6. | Magnesiumstearat | 2 mg |
| | | 200 mg |

Der Wirkstoff wird mit Milchzucker homogen vermischt. Man siebt, mischt dann eine Mischung aus mikrokristalliner Cellulose, Maisstärke und Natrium-carboxymethylstärke zu und vermengt mit dem Magnesiumstearat. Die so erhaltene pressfertige Mischung wird zu Tabletten geeigneter Grösse mit Bruchrille verarbeitet.

## Patentansprüche

**Patentansprüche für folgende Vertragsstaaten : BE, CH, DE, FR, IT, LI, LU, NL, SE**

1. Benzazecin-Derivate der allgemeinen Formel

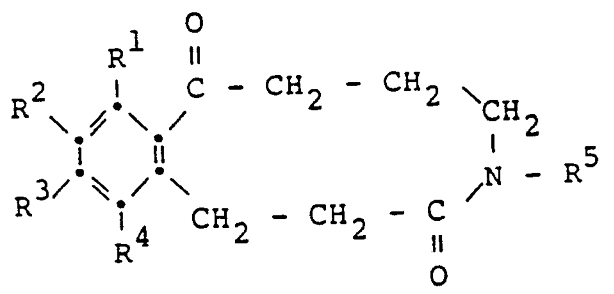

I

worin $R^1$ und $R^2$ je Wasserstoff oder Chlor, $R^3$ Wasserstoff, Fluor, Chlor, Brom oder Methoxy, $R^4$ Wasserstoff, Chlor oder Methoxy und $R^5$ Wasserstoff, Acetyl, Propionyl, Benzoyl, Chlorbenzoyl, Methoxybenzoyl oder Phenylacetyl bedeuten, mit der Massgabe, dass 2 oder 3 der Symbole $R^1$ bis $R^4$ Wasserstoff bedeuten.

2. Benzazecin-Derivate gemäss Anspruch 1, worin $R^1$ Chlor und $R^2$, $R^3$ and $R^4$ je Wasserstoff bedeuten.

3. Benzazecin-Derivate gemäss Anspruch 1, worin $R^2$ Chlor und $R^1$, $R^3$ und $R^4$ je Wasserstoff bedeuten.

4. Benzazecin-Derivate gemäss Anspruch 1, worin $R^3$ Fluor, Chlor, Brom oder Methoxy und $R^1$, $R^2$ und $R^4$ je Wasserstoff bedeuten.

5. Benzazecin-Derivate gemäss Anspruch 1, worin $R^4$ Chlor oder Methoxy und $R^1$, $R^2$ und $R^3$ je Wasserstoff bedeuten.

6. Benzazecin-Derivate gemäss Anspruch 1, worin $R^1$ und $R^3$ je Chlor und $R^2$ und $R^4$ je Wasserstoff bedeuten.

7. Benzazecin-Derivate gemäss einem der Ansprüche 1 bis 6, worin $R^5$ Acetyl bedeutet.

8. 4-Acetyl-9-chlor-1,2,4,5,6,7-hexahydro-4-benzazecin-3,8-dion.

9. 4-Acetyl-11-chlor-1,2,4,5,6,7-hexahydro-4-benzazecin-3,8-dion.

**10.** 4-Acetyl-11-fluor-1,2,4,5,6,7-hexahydro-4-benzazecin-3,8-dion.

**11.** 4-Acetyl-9,11-dichlor-1,2,4,5,6,7-hexahydro-4-benzazecin-3,8-dion.

**12.** 4-Acetyl-11-methoxy-1,2,4,5,6,7-hexahydro-4-benzazecin-3,8-dion.

**13.** 11-Chlor-1,2,4,5,6,7-hexahydro-4-propionyl-4-benzazecin-3,8-dion.

**14.** 4-Benzoyl-11-chlor-1,2,4,5,6,7-hexahydro-4-benzazecin-3,8-dion.

**15.** 4-(o-Methoxybenzoyl)-11-chlor-1,2,4,5,6,7-hexahydro-4-benzazecin-3,8-dion.

**16.** 4-(m-Methoxybenzoyl)-11-chlor-1,2,4,5,6,7-hexahydro-4-benzazecin-3,8-dion.

**17.** 4-(p-Methoxybenzyol)-11-chlor-1,2,4,5,6,7-hexahydro-4-benzazecin-3,8-dion.

**18.** 11-Chlor-4-(p-chlorbenzoyl)-1,2,4,5,6,7-hexahydro-4-benzazecin-3,8-dion.

**19.** 4-Acetyl-1,2,4,5,6,7-hexahydro-12-methoxy-4-benzazecin-3,8-dion.

**20.** 9-Chlor-1,2,4,5,6,7-hexahydro-4-benzazecin-3,8-dion;
4-Acetyl-10-chlor-1,2,4,5,6,7-hexahydro-4-benzazecin-3,8-dion;
11-Chlor-1,2,4,5,6,7-hexahydro-4-(phenylacetyl)-4-benzazecin-3,8-dion;
11-Chlor-1,2,4,5,6,7-hexahydro-4-benzazecin-3,8-dion;
4-Acetyl-11-brom-1,2,4,5,6,7-hexahydro-4-benzazecin-3,8-dion;und
4-Acetyl-12-chlor-1,2,4,5,6,7-hexahydro-4-benzazecin-3,8-dion.

**21.** Benzochinolin-Derivate der allgemeinen Formel

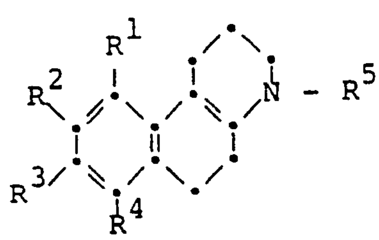

II

worin $R^1$, $R^2$, $R^3$, $R^4$ und $R^5$ die in Anspruch 1 angegebene Bedeutung besitzen.

**22.** 4-Acetyl-8-chlor-1,2,3,4,5,6-hexahydrobenzo[f]chinolin.

**23.** 4-Acetyl-10-chlor-1,2,3,4,5,6-hexahydrobenzo[f]chinolin.

**24.** 8-Chlor-4-(p-chlorbenzoyl)-1,2,3,4,5,6-hexahydrobenzo[f]chinolin.

**25.** Benzazecin-Derivate der allgemeinen Formel

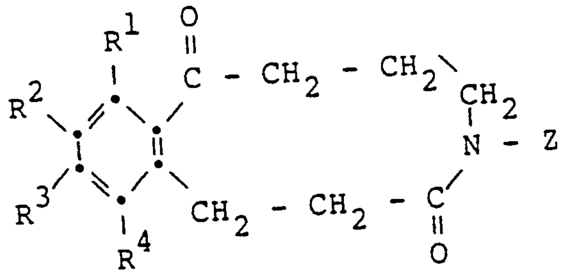

III

worin $R^1$, $R^2$, $R^3$ und $R^4$ die in Anspruch 1 angegebene Bedeutung besitzen und Z eine abspaltbare Gruppe, nicht jedoch Acetyl, Propionyl, Benzoyl, Chlorbenzoyl, Methoxybenzoyl oder Phenylacetyl bedeutet.

**26.** Verbindungen gemäss einem der Ansprüche 1 bis 20 und 22 bis 24 zur Anwendung als therapeutische Wirkstoffe.

**27.** Verbindungen gemäss einem der Ansprüche 1 bis 20 und 22 bis 24 zur Anwendung als der cerebralen Insuffizienz entgegenwirkende bzw. cognitive Funktionen verbessernde therapeutische Wirkstoffe.

**28.** Verfahren zur Herstellung von Benzazecin-Derivaten gemäss einem der Ansprüche 1 bis 20, dadurch gekennzeichnet, dass man

a) ein Benzochinolin-Derivat der allgemeinen Formel

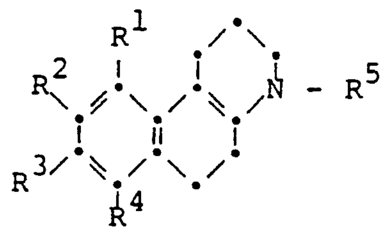

II

worin $R^1$, $R^2$, $R^3$, $R^4$ und $R^5$ die in Anspruch 1 angegebene Bedeutung besitzen,
oxydiert oder

b) von einem Benzazecin-Derivat der allgemeinen Formel

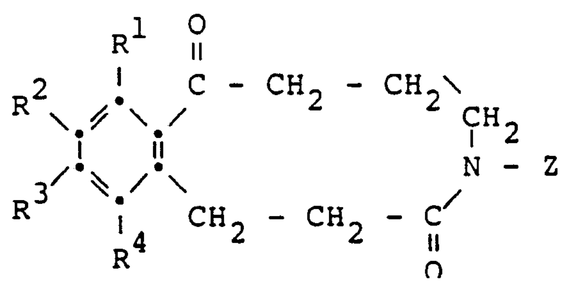

III

worin $R^1$, $R^2$, $R^3$ und $R^4$ die in Anspruch 1 angegebene Bedeutung besitzen und Z eine abspaltbare Gruppe bedeutet,

die mit Z bezeichnete Gruppe entfernt.

**29.** Verfahren zur Herstellung von Benzochinolin-Derivaten gemäss einem der Ansprüche 21 bis 24, dadurch gekennzeichnet, dass man

a) ein Benzochinolinon-Derivat der allgemeinen Formel

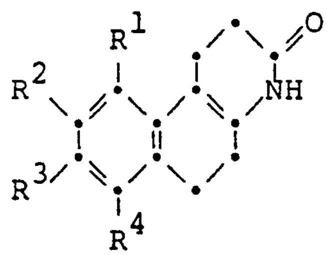

IV

worin $R^1$, $R^2$, $R^3$ und $R^4$ die in Anspruch 1 angegebene Bedeutung besitzen, reduziert oder

b) eine Verbindung der allgemeinen Formel

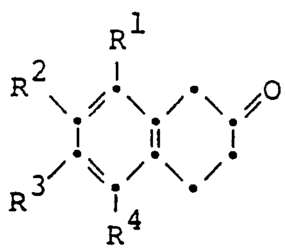

V

worin $R^1$, $R^2$, $R^3$ und $R^4$ die in Anspruch 1 angegebene Bedeutung besitzen, in Gegenwart einer starken Base mit einer Verbindung der allgemeinen Formel

$X-(CH_2)_3-NH_2$ VI

worin X eine Abgangsgruppe bedeutet, umsetzt und erwünschtenfalls die erhaltene Verbindung der allgemeinen Formel II, worin $R^5$ Wasserstoff bedeutet, durch Acetyl, Propionyl, Benzoyl, Chlorbenzoyl, Methoxybenzoyl oder Phenylacetyl N-substituiert.

30. Arzneimittel, enthaltend eine Verbindung gemäss einem der Ansprüche 1 bis 20 und 22 bis 24 und ein therapeutisch inertes Excipiens.

31. Der cerebralen Insuffizienz entgegenwirkendes bzw. cognitive Funktionen verbesserndes Arzneimittel, enthaltend eine Verbindung gemäss einem der Ansprüche 1 bis 20 und 22 bis 24 und ein therapeutisch inertes Excipiens.

32. Verbindungen gemäss einem der Ansprüche 1 bis 20 und 22 bis 24 zur Bekämpfung bzw. Verhütung von Krankheiten bzw. bei der Verbesserung der Gesundheit.

33. Verbindungen gemäss einem der Ansprüche 1 bis 20 und 22 bis 24 zur Bekämpfung bzw. Verhütung der cerebralen Insuffizienz bzw. bei der Verbesserung cognitiver Funktionen.

34. Verwendung von Verbindungen gemäss einem der Ansprüche 1 bis 20 und 22 bis 24 zur Herstellung von Arzneimitteln für die Bekämpfung bzw. Verhütung der cerebralen Insuffizienz bzw. für die Verbesserung cognitiver Funktionen.

**Patentansprüche für folgende Vertragsstaaten : AT, ES**

1. Verfahren zur Herstellung von Benzazecin-Derivate der allgemeinen Formel

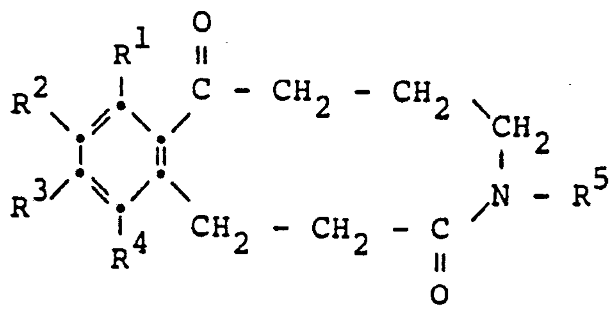

I

worin $R^1$ und $R^2$ je Wasserstoff oder Chlor, $R^3$ Wasserstoff, Fluor, Chlor, Brom oder Methoxy, $R^4$ Wasserstoff, Chlor oder Methoxy und $R^5$ Wasserstoff, Acetyl, Propionyl, Benzoyl, Chlorbenzoyl, Methoxybenzoyl oder Phenylacetyl bedeuten, mit der Massgabe, dass 2 oder 3 der Symbole $R^1$ bis $R^4$ Wasserstoff bedeuten,
dadurch gekennzeichnet, dass man

a) ein Benzochinolin-Derivat der allgemeinen Formel

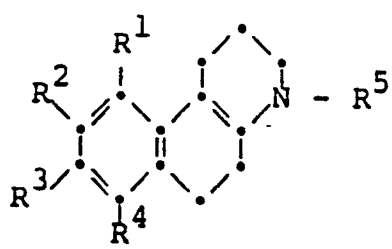

II

worin $R^1$, $R^2$, $R^3$, $R^4$ und $R^5$ obige Bedeutung besitzen,
oxydiert oder

b) von einem Benzazecin-Derivat der allgemeinen Formel

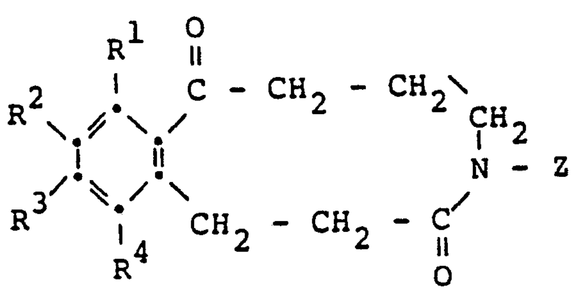

III

worin $R^1$, $R^2$, $R^3$ und $R^4$ obige Bedeutung besitzen und Z eine abspaltbare Gruppe bedeutet,
die mit Z bezeichnete Gruppe entfernt.

2. Verfahren gemäss Anspruch 1, dadurch gekennzeichnet, dass $R^1$ Chlor und $R^2$, $R^3$ und $R^4$ je Wasserstoff bedeuten.

3. Verfahren gemäss Anspruch 1, dadurch gekennzeichnet, dass $R^2$ Chlor und $R^1$, $R^3$ und $R^4$ je Wasserstoff bedeuten.

4. Verfahren gemäss Anspruch 1, dadurch gekennzeichnet, dass $R^3$ Fluor, Chlor, Brom oder Methoxy und $R^1$, $R^2$ und $R^4$ je Wasserstoff bedeuten.

5. Verfahren gemäss Anspruch 1, dadurch gekennzeichnet, dass $R^4$ Chlor oder Methoxy und $R^1$, $R^2$ und $R^3$ je Wasserstoff bedeuten.

6. Verfahren gemäss Anspruch 1, dadurch gekennzeichnet, dass $R^1$ und $R^3$ je Chlor und $R^2$ und $R^4$ je Wasserstoff bedeuten.

7. Verfahren gemäss einem der Ansprüche 1 bis 6, dadurch gekennzeichnet, dass $R^5$ Acetyl bedeutet.

8. Verfahren gemäss Anspruch 1, dadurch gekennzeichnet, dass man 4-Acetyl-9-chlor-1,2,4,5,6,7-hexahydro-4-benzazecin-3,8-dion herstellt.

9. Verfahren gemäss Anspruch 1, dadurch gekennzeichnet, dass man 4-Acetyl-11-chlor-1,2,4,5,6,7-hexahydro-4-benzazecin-3,8-dion herstellt.

10. Verfahren gemäss Anspruch 1, dadurch gekennzeichnet, dass man 4-Acetyl-11-fluor-1,2,4,5,6,7-hexahydro-4-benzazecin-3,8-dion herstellt.

11. Verfahren gemäss Anspruch 1, dadurch gekennzeichnet, dass man 4-Acetyl-9,11-dichlor-1,2,4,5,6,7-hexahydro-4-benzazecin-3,8-dion herstellt.

12. Verfahren gemäss Anspruch 1, dadurch gekennzeichnet, dass man 4-Acetyl-11-methoxy-1,2,4,5,6,7-hexahydro-4-benzazecin-3,8-dion herstellt.

13. Verfahren gemäss Anspruch 1, dadurch gekennzeichnet, dass man 11-Chlor-1,2,4,5,6,7-hexahydro-4-propionyl-4-benzazecin-3,8-dion herstellt.

14. Verfahren gemäss Anspruch 1, dadurch gekennzeichnet, dass man 4-Benzoyl-11-chlor-1,2,4,5,6,7-hexahydro-4-benzazecin-3,8-dion herstellt.

15. Verfahren gemäss Anspruch 1, dadurch gekennzeichnet, dass man 4-(o-Methoxybenzoyl)-11-chlor-1,2,4,5,6,7-hexahydro-4-benzazecin-3,8-dion herstellt.

16. Verfahren gemäss Anspruch 1, dadurch gekennzeichnet, dass man 4-(m-Methoxybenzoyl)-11-chlor-1,2,4,5,6,7-hexahydro-4-benzazecin-3,8-dion herstellt.

17. Verfahren gemäss Anspruch 1, dadurch gekennzeichnet, dass man 4-(p-Methoxybenzyol)-11-chlor-1,2,4,5,6,7-hexahydro-4-benzazecin-3,8-dion herstellt.

18. Verfahren gemäss Anspruch 1, dadurch gekennzeichnet, dass man 11-Chlor-4-(p-chlorbenzoyl)-1,2,4,5,6,7-hexahydro-4-benzazecin-3,8-dion herstellt.

19. Verfahren gemäss Anspruch 1, dadurch gekennzeichnet, dass man 4-Acetyl-1,2,4,5,6,7-hexahydro-12-methoxy-4-benzazecin-3,8-dion herstellt.

20. Verfahren zur Herstellung von Benzochinolin-Derivaten der in Anspruch 1 definierten allgemeinen Formel II, dadurch gekennzeichnet, dass man

a) ein Benzochinolinon-Derivat der allgemeinen Formel

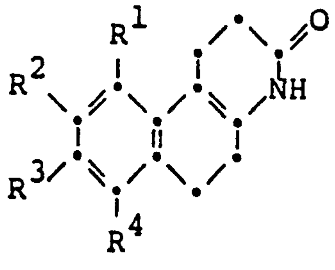

IV

worin $R^1$, $R^2$, $R^3$ und $R^4$ die in Anspruch 1 angegebene Bedeutung besitzen, reduziert oder

b) eine Verbindung der allgemeinen Formel

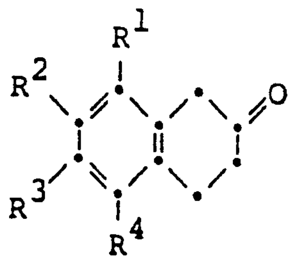

V

worin $R^1$, $R^2$, $R^3$ und $R^4$ die in Anspruch 1 angegebene Bedeutung besitzen, in Gegenwart einer starken Base mit einer Verbindung der allgemeinen Formel

$X-(CH_2)_3-NH_2$ VI

worin X eine Abgangsgruppe bedeutet,
umsetzt und erwünschtenfalls die erhaltene Verbindung der allgemeinen Formel II, worin $R^5$ Wasserstoff bedeutet, durch Acetyl, Propionyl, Benzoyl, Chlorbenzoyl, Methoxybenzoyl oder Phenylacetyl N-substituiert.

**21.** Verfahren gemäss Anspruch 20, dadurch gekennzeichnet, dass man 4-Acetyl-8-chlor-1,2,3,4,5,6-hexahydrobenzo[f]chinolin herstellt.

**22.** Verfahren gemäss Anspruch 20, dadurch gekennzeichnet, dass man 4-Acetyl-10-chlor-1,2,3,4,5,6-hexahydrobenzo[f]chinolin herstellt.

**23.** Verfahren gemäss Anspruch 20, dadurch gekennzeichnet, dass man 8-Chlor-4-(p-chlorbenzoyl)-1,2,3,4,5,6-hexahydrobenzo[f]chinolin herstellt.

**24.** Verwendung von Verbindungen der in Anspruch 1 definierten allgemeinen Formel I sowie von 4-Acetyl-8-chlor-1,2,3,4,5,6-hexahydrobenzo[f]chinolin, 4-Acetyl-10-chlor-1,2,3,4,5,6-hexahydrobenzo[f]chinolin und von 8-Chlor-4-(p-chlorbenzoyl)-1,2,3,4,5,6-hexahydro-benzo[f]chinolin zur Herstellung von Arzneimitteln für die Bekämpfung bzw. Verhütung der cerebralen Insuffizienz bzw. für die Verbesserung cognitiver Funktionen.

**25.** Benzazecin-Derivate der allgemeinen Formel

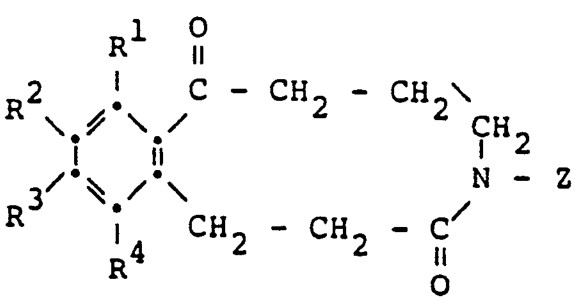

III

worin $R^1$, $R^2$, $R^3$ und $R^4$ die in Anspruch 1 angegebene Bedeutung besitzen und Z eine abspaltbare Gruppe, nicht jedoch Acetyl, Propionyl, Benzoyl, Chlorbenzoyl, Methoxybenzoyl oder Phenylacetyl bedeutet.

**26.** Benzochinolin-Derivate der allgemeinen Formel

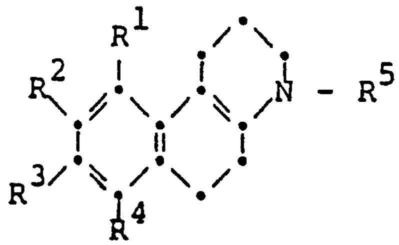

II

worin $R^1$, $R^2$, $R^3$, $R^4$ und $R^5$ die in Anspruch 1 angegebene Bedeutung besitzen, nicht jedoch 4-Acetyl-8-chlor-1,2,3,4,5,6-hexahydrobenzo-[f]chinolin, 4-Acetyl-10-chlor-1,2,3,4,5,6-hexahydrobenzo-[f]chinolin, und 8-Chlor-4-(p-chlorbenzoyl)-1,2,3,4,5,6--hexahydro-benzo[f]chinolin.

**Patentansprüche für folgende Vertragsstaat : GR**

**1.** Verfahren zur Herstellung von Benzazecin-Derivate der allgemeinen Formel

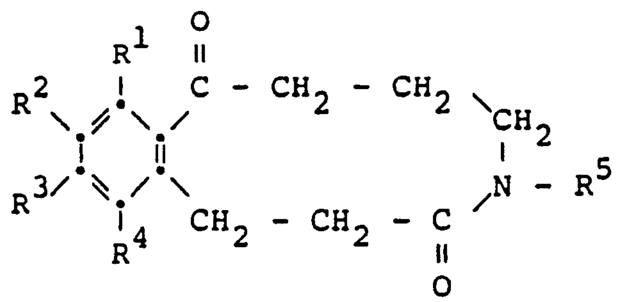

I

worin $R^1$ und $R^2$ je Wasserstoff oder Chlor, $R^3$ Wasserstoff, Fluor, Chlor, Brom oder Methoxy, $R^4$ Wasserstoff, Chlor oder Methoxy und $R^5$ Wasserstoff, Acetyl, Propionyl, Benzoyl, Chlorbenzoyl, Methoxybenzoyl oder Phenylacetyl bedeuten, mit der Massgabe, dass 2 oder 3 der Symbole $R^1$ bis $R^4$ Wasserstoff bedeuten,
dadurch gekennzeichnet, dass Man
a) ein Benzochinolin-Derivate der allgemeinen Formel

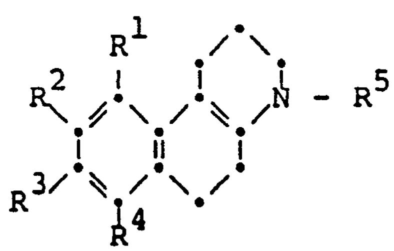

II

worin $R^1$, $R^2$, $R^3$, $R^4$ und $R^5$ obige Bedeutung besitzen,
oxydiert oder
b) von einem Benzazecin-Derivat der allgemeinen Formel

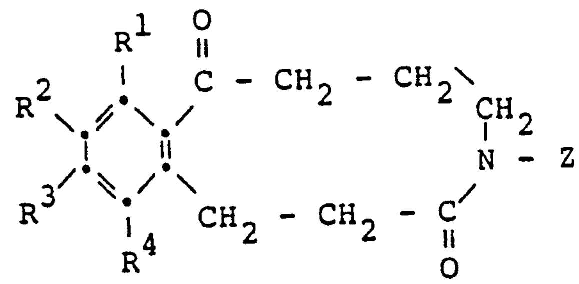

III

worin $R^1$, $R^2$, $R^3$ und $R^4$ obige Bedeutung besitzen und Z eine abspaltbare Gruppe bedeutet, die mit Z bezeichnete Gruppe entfernt.

2. Verfahren gemäss Anspruch 1, dadurch gekennzeichnet, dass $R^1$ Chlor und $R^2$, $R^3$ und $R^4$ je Wasserstoff bedeuten.

3. Verfahren gemäss Anspruch 1, dadurch gekennzeichnet, dass $R^2$ Chlor und $R^1$, $R^3$ und $R^4$ je Wasserstoff bedeuten.

4. Verfahren gemäss Anspruch 1, dadurch gekennzeichnet, dass $R^3$ Fluor, Chlor, Brom oder Methoxy und $R^1$, $R^2$ und $R^4$ je Wasserstoff bedeuten.

5. Verfahren gemäss Anspruch 1, dadurch gekennzeichnet, dass $R^4$ Chlor oder Methoxy und $R^1$, $R^2$ und $R^3$ je Wasserstoff bedeuten.

6. Verfahren gemäss Anspruch 1, dadurch gekennzeichnet, dass $R^1$, und $R^3$ je Chlor und $R^2$ und $R^4$ je Wasserstoff bedeuten.

7. Verfahren gemäss einem der Ansprüche 1 bis 6, dadurch gekennzeichnet, dass $R^5$ Acetyl bedeutet.

8. Verfahren gemäss Anspruch 1, dadurch gekennzeichnet, dass man 4-Acetyl-9-chlor-1,2,4,5,6,7-hexahydro-4-benzazecin-3,8-dion herstellt.

9. Verfahren gemäss Anspruch 1, dadurch gekennzeichnet, dass man 4-Acetyl-11-chlor-1,2,4,5,6,7-hexahydro-4-benzazecin-3,8-dion herstellt.

10. Verfahren gemäss Anspruch 1, dadurch gekennzeichnet, dass man 4-Acetyl-11-fluor-1,2,4,5,6,7-hexahydro-4-benzazecin-3,8-dion herstellt.

11. Verfahren gemäss Anspruch 1, dadurch gekennzeichnet, dass man 4-Acetyl-9,11-dichlor-1,2,4,5,6,7-hexahydro-4-benzazecin-3,8-dion herstellt.

12. Verfahren gemäss Anspruch 1, dadurch gekennzeichnet, dass man 4-Acetyl-11-methoxy-1,2,4,5,6,7-hexahydro-4-benzazecin-3,8-dion herstellt.

13. Verfahren gemäss Anspruch 1, dadurch gekennzeichnet, dass man 11-Chlor-1,2,4,5,6,7-hexahydro-4-propionyl-4-bezazecin-3,8-dion herstellt.

14. Verfahren gemäss Anspruch 1, dadurch gekennzeichnet, dass man 4-Benzoyl-11-chlor-1,2,4,5,6,7-hexahydro-4-benzazecin-3,8-dion herstellt.

15. Verfahren gemäss Anspruch 1, dadurch gekennzeichnet, dass man 4-(o-Methoxybenzoyl)-11-chlor-1,2,4,5,6,7-hexahydro-4-benzazecin-3,8-dion herstellt.

16. Verfahren gemäss Anspruch 1, dadurch gekennzeichnet, dass man 4-(m-Methoxybenzoyl)-11-chlor-1,2,4,5,6,7-hexahydro-4-benzazecin-3,8-dion herstellt.

17. Verfahren gemäss Anspruch 1, dadurch gekennzeichnet, dass man 4-(p-Methoxybenzyol)-11-chlor-1,2,4,5,6,7-hexahydro-4-benzazecin-3,8-dion herstellt.

18. Verfahren gemäss Anspruch 1, dadurch gekennzeichnet, dass man 11-Chlor-4-(p-chlorbenzoyl)-1,2,4,5,6,7-hexahydro-4-benzazecin-3,8-dion herstellt.

19. Verfahren gemäss Anspruch 1, dadurch gekennzeichnet, dass man 4-Acetyl-1,2,4,5,6,7-hexahydro-12-methoxy-4-benzazecin-3,8-dion herstellt.

20. Verfahren zur Herstellung von Benzochinolin-Derivaten der in Anspruch 1 definierten allgemeinen Formel II, dadurch gekennzeichnet, dass man
a) ein Benzochinolinon-Derivat der allgemeinen Formel

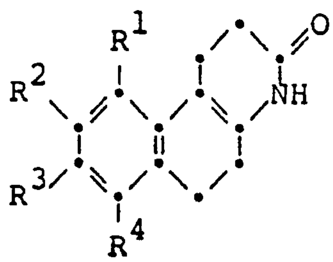

IV

worin $R^1$, $R^2$, $R^3$ und $R^4$ die in Anspruch 1 angegebene Bedeutung besitzen, reduziert oder
b) eine Verbindung der allgemeinen Formel

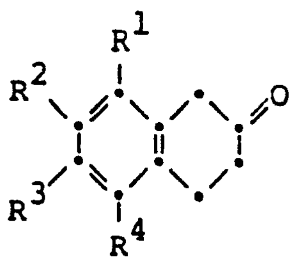

V

worin $R^1$, $R^2$, $R^3$ und $R^4$ die in Anspruch 1 angegebene Bedeutung besitzen, in Gegenwart einer starken Base mit einer Verbindung der allgemeinen Formel

$X\text{-}(CH_2)_3\text{-}NH_3$ VI

worin X eine Abgangsgruppe bedeutet, umsetzt und erwünschtenfalls die erhaltene Verbindung der allgemeinen Formel II, worin $R^5$ Wasserstoff bedeutet, durch Acetyl, Propionyl, Benzoyl, Chlorbenzoyl, Methoxybenzoyl oder Phenyacetyl N-substituiert.

21. Verfahren gemäss Anspruch 20, dadurch gekennzeichnet, dass man 4-Acetyl-8-chlor-1,2,3,4,5,6-hexahydrobenzo[f]chinolin herstellt.

22. Verfahren gemäss Anspruch 20, dadurch gekennzeichnet, dass man 4-Acetyl-10-chlor-1,2,3,4,5,6-hexahydrobenzo[f]chinolin herstellt.

23. Verfahren gemäss Anspruch 20, dadurch gekennzeichnet, dass man 8-Chlor-4-(p-chlorbenzoyl)-1,2,3,4,5,6-hexahydrobenzo[f]chinolin herstellt.

24. Verwendung von Verbindungen der in Anspruch 1 definierten allgemeinen Formel I sowie von 4-Acetyl-8-chlor-1,2,3,4,5,6-hexahydrobenzo[f]chinolin, 4-Acetyl-10-chlor-1,2,3,4,5,6-hexahydrobenzo[f]chinolin und von 8-Chlor-4-(p-chlorbenzoyl)-1,2,3,4,5,6-hexahydro-benzo[f]chinolin zur Herstellung von Arzneimitteln für die Bekämpfung bzw. Verhütung der cerebralen Insuffizienz bzw. für die Verbesserung cognitiver Funktionen.

**Claims**

**Claims for the following Contracting States : BE, CH, DE, FR, IT, LI, LU, NL, SE**

1. Benzazecine derivatives of the general formula

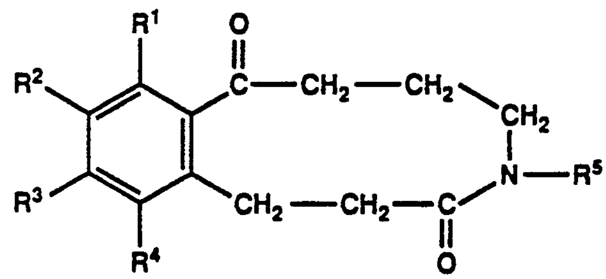

I

wherein $R^1$ and $R^2$ each signify hydrogen or chlorine, $R^3$ signifies hydrogen, fluorine, chlorine, bromine or methoxy, $R^4$ signifies hydrogen, chlorine or methoxy and $R^5$ signifies hydrogen, acetyl, propionyl, benzoyl, chlorobenzoyl, methoxybenzoyl or phenylacetyl, with the proviso that 2 or 3 of the symbols $R^1$ to $R^4$ signify hydrogen.

2. Benzazecine derivatives in accordance with claim 1, wherein $R^1$ signifies chlorine and $R^2$, $R^3$ and $R^4$ each signify hydrogen.

3. Benzazecine derivatives in accordance with claim 1, wherein $R^2$ signifies chlorine and $R^1$, $R^3$ and $R^4$ each signify hydrogen.

4. Benzazecine derivatives in accordance with claim 1, wherein $R^3$ signifies fluorine, chlorine, bromine or methoxy and $R^1$, $R^2$ and $R^4$ each signify hydrogen.

5. Benzazecine derivatives in accordance with claim 1, wherein $R^4$ signifies chlorine or methoxy and $R^1$, $R^2$ and $R^3$ each signify hydrogen.

6. Benzazecine derivatives in accordance with claim 1, wherein $R^1$ and $R^3$ each signify chlorine and $R^2$ and $R^4$ each signify hydrogen.

7. Benzazecine derivatives in accordance with any one of claims 1 to 6, wherein $R^5$ signifies acetyl.

8. 4-Acetyl-9-chloro-1,2,4,5,6,7-hexahydro-4-benzazecine-3,8-dione.

9. 4-Acetyl-11-chloro-1,2,4,5,6,7-hexahydro-4-benzazecine-3,8-dione.

10. 4-Acetyl-11-fluoro-1,2,4,5,6,7-hexahydro-4-benzazecine-3,8-dione.

11. 4-Acetyl-9,11-dichloro-1,2,4,5,6,7-hexahydro-4-benzazecine-3,8-dione.

12. 4-Acetyl-11-methoxy-1,2,4,5,6,7-hexahydro-4-benzazecine-3,8-dione.

13. 11-Chloro-1,2,4,5,6,7-hexahydro-4-propionyl-4-benzazecine-3,8-dione.

14. 4-Benzoyl-11-chloro-1,2,4,5,6,7-hexahydro-4-benzazecine-3,8-dione.

**15.** 4-(o-Methoxybenzoyl)-11-chloro-1,2,4,5,6,7-hexahydro-4-benzazecine-3,8-dione.

**16.** 4-(m-Methoxybenzoyl)-11-chloro-1,2,4,5,6,7-hexahydro-4-benzazecine-3,8-dione.

**17.** 4-(p-Methoxybenzoyl)-11-chloro-1,2,4,5,6,7-hexahydro-4-benzazecine-3,8-dione

**18.** 11-Chloro-4-(p-chlorobenzoyl)-1,2,4,5,6,7-hexahydro-4-benzazecine-3,8-dione.

**19.** 4-Acetyl-1,2,4,5,6,7-hexahydro-12-methoxy-4-benzazecine-3,8-dione.

**20.** 9-Chloro-1,2,4,5,6,7-hexahydro-4-benzazecine-3,8-dione;
4-acetyl-10-chloro-1,2,4,5,6,7-hexahydro-4-benzazecine-3,8-dione;
11-chloro-1,2,4,5,6,7-hexahydro-4-(phenylacetyl)-4-benzazecine-3,8-dione;
11-chloro-1,2,4,5,6,7-hexahydro-4-benzazecine-3,8-dione;
4-acetyl-11-bromo-1,2,4,5,6,7-hexahydro-4-benzazecine-3,8-dione; and
4-acetyl-12-chloro-1,2,4,5,6,7-hexahydro-4-benzazecine-3,8-dione.

**21.** Benzoquinoline derivatives of the general formula

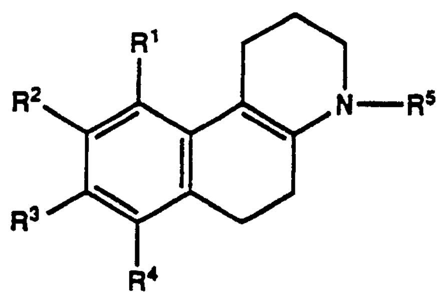

II

wherein $R^1$, $R^2$, $R^3$, $R^4$ and $R^5$ have the significance given in claim 1.

**22.** 4-Acetyl-8-chloro-1,2,3,4,5,6-hexahyarobenzo[f]-quinoline.

**23.** 4-Acetyl-10-chloro-1,2,3,4,5,6-hexahydrobenzo[f]-quinoline.

**24.** 8-Chloro-4-(p-chlorobenzoyl)-1,2,3,4,5,6-hexahydrobenzo[f]quinoline.

**25.** Benzazecine derivatives of the general formula

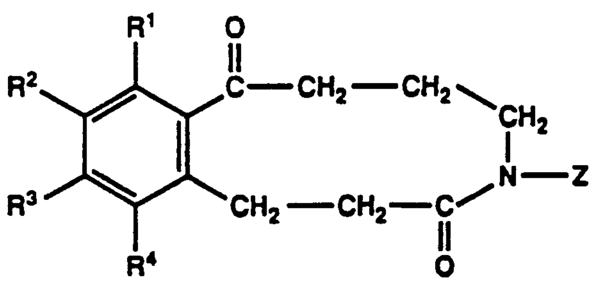

III

wherein $R^1$, $R^2$, $R^3$ and $R^4$ have the significance given in claim 1 and Z signifies a cleavable group, but not acetyl, propionyl, benzoyl, chlorobenzoyl, methoxybenzoyl or phenylacetyl.

**26.** Compounds in accordance with any one of claims 1 to 20 and 22 to 24 for use as therapeutically active substances.

27. Compounds in accordance with any one of claims 1 to 20 and 22 to 24 for use as therapeutically active substances counteracting cerebral insufficiency or improving cognitive functions.

28. A process for the manufacture of benzazecine derivatives in accordance with any one of claims 1 to 20, characterized by

a) oxidizing a benzoquinoline derivative of the general formula

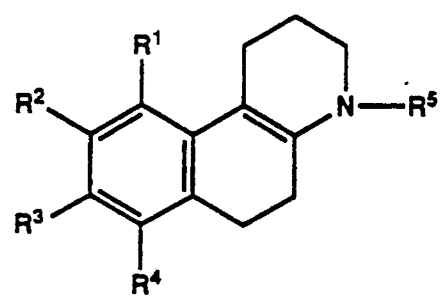

II

wherein $R^1$, $R^2$, $R^3$, $R^4$ and $R^5$ have the significance given in claim 1,

or

b) removing the group denoted by Z from a benzazecine derivative of the general formula

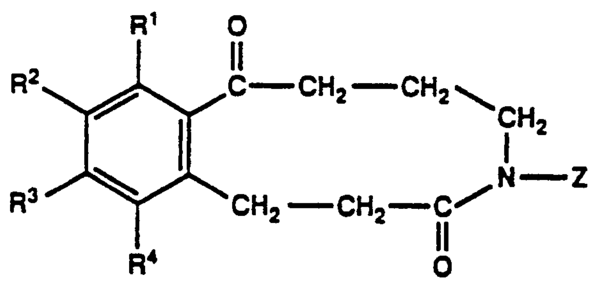

III

wherein $R^1$, $R^2$, $R^3$, and $R^4$ have the significance given in claim 1 and Z signifies a cleavable group.

29. A process for the preparation of benzoquinoline derivatives in accordance with any one of claims 21 to 24, characterized by

a) reducing a benzoquinolinone derivative of the general formula

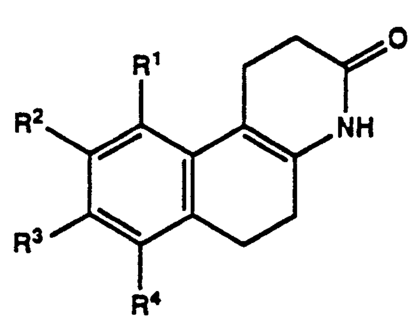

IV

wherein $R^1$, $R^2$, $R^3$ and $R^4$ have the significance given in claim 1,

or

b) reacting a compound of the general formula

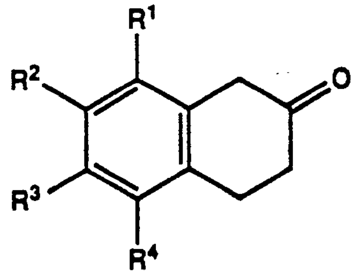

V

wherein $R^1$, $R^2$, $R^3$ and $R^4$ have the significance given in claim 1, in the presence of a strong base with a compound of the general formula

$X-(CH_2)_3-NH_2$ VI

wherein X signifies a leaving group, and, if desired, N-substituting the obtained compound of general formula II in which $R^5$ signifies hydrogen by acetyl, propionyl, benzoyl, chlorobenzoyl, methoxybenzoyl or phenylacetyl.

**30.** A medicament containing a compound in accordance with any one of claims 1 to 20 and 22 to 24 and a therapeutically inert excipient.

**31.** A medicament counteracting cerebral insufficiency or improving cognitive functions, containing a compound in accordance with any one of claims 1 to 20 and 22 to 24 and a therapeutically inert excipient.

**32.** Compounds in accordance with any one of claims 1 to 20 and 22 to 24 for the control or prevention of illnesses or in the improvement of health.

**33.** Compounds in accordance with any one of claims 1 to 20 and 22 to 24 for the control or prevention of cerebral insufficiency or in the improvement of cognitive functions.

**34.** The use of compounds in accordance with any one of claims 1 to 20 and 22 to 24 for the manufacture of medicaments for the control or prevention of cerebral insufficiency or for the improvement of cognitive functions

**Claims for the following Contracting States : AT, ES**

**1.** A process for the manufacture of benzazecine derivatives of the general formula

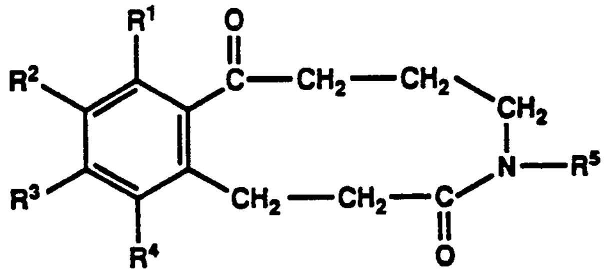

I

wherein $R^1$ and $R^2$ each signify hydrogen or chlorine, $R^3$ signifies hydrogen, fluorine, chlorine, bromine or methoxy, $R^4$ signifies hydrogen, chlorine or methoxy and $R^5$ signifies hydrogen, acetyl, propionyl, benzoyl, chlorobenzoyl, methoxybenzoyl or phenylacetyl, with the proviso that 2 or 3 of the symbols $R^1$ to $R^4$ signify hydrogen, characterized by

a) oxidizing a benzoquinoline derivative of the general formula

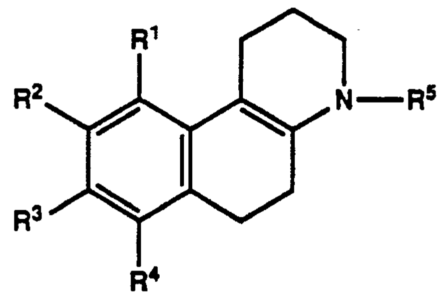

II

wherein $R^1$, $R^2$, $R^3$, $R^4$ and $R^5$ have the above significance,
or
b) removing the group denoted by Z from a benzazecine derivative of the general formula

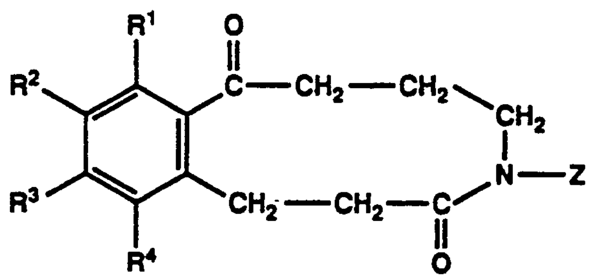

III

wherein $R^1$, $R^2$, $R^3$, and $R^4$ have the above significance and Z signifies a cleavable group.

2. A process in accordance with claim 1, characterized in that $R^1$ signifies chlorine and $R^2$, $R^3$ and $R^4$ each signify hydrogen.

3. A process in accordance with claim 1, characterized in that $R^2$ signifies chlorine and $R^1$, $R^3$ and $R^4$ each signify hydrogen.

4. A process in accordance with claim 1, characterized in that $R^3$ signifies fluorine, chlorine, bromine or methoxy and $R^1$, $R^2$ and $R^4$ each signify hydrogen.

5. A process in accordance with claim 1, characterized in that $R^4$ signifies chlorine or methoxy and $R^1$, $R^2$ and $R^3$ each signify hydrogen.

6. A process in accordance with claim 1, characterized in that $R^1$ and $R^3$ each signify chlorine and $R^2$ and $R^4$ each signify hydrogen.

7. A process in accordance with any one of claims 1 to 6, characterized in that $R^5$ signifies acetyl.

8. A process in accordance with claim 1, characterized in that 4-acetyl-9-chloro-1,2,4,5,6,7-hexahydro-4-benzazecine-3,8-dione is manufactured.

9. A process in accordance with claim 1, characterized in that 4-acetyl-11-chloro-1,2,4,5,6,7-hexahydro-4-benzazecine-3,8-dione is manufactured.

10. A process in accordance with claim 1, characterized in that 4-acetyl-11-fluoro-1,2,4,5,6,7-hexahydro-4-benzazecine-3,8-dione is manufactured.

11. A process in accordance with claim 1, characterized in that 4-acetyl-9,11-dichloro-1,2,4,5,6,7-hexahydro-4-benzazecine-3,8-dione is manufactured.

**12.** A process in accordance with claim 1, characterized in that 4-acetyl-11-methoxy-1,2,4,5,6,7-hexahydro-4-benzazecine-3,8-dione is manufactured.

**13.** A process in accordance with claim 1, characterized in that 11-chloro-1,2,4,5,6,7-hexahydro-4-propionyl-4-benzazecine-3,8-dione is manufactured.

**14.** A process in accordance with claim 1, characterized in that 4-benzoyl-11-chloro-1,2,4,5,6,7-hexahydro-4-benzazecine-3,8-dione is manufactured

**15.** A process in accordance with claim 1, characterized in that 4-(o-methoxybenzoyl)-11-chloro-1,2,4,5,6,7-hexahydro-4-benzazecine-3,8-dione is manufactured.

**16.** A process in accordance with claim 1, characterized in that 4-(m-methoxybenzoyl)-11-chloro-1,2,4,5,6,7-hexahydro-4-benzazecine-3,8-dione is manufactured.

**17.** A process in accordance with claim 1, characterized in that 4-(p-methoxybenzoyl)-11-chloro-1,2,4,5,6,7-hexa-hydro-4-benzazecine-3,8-dione is manufactured.

**18.** A process in accordance with claim 1, characterized in that 11-chloro-4-(p-chlorobenzoyl)-1,2,4,5,6,7-hexahydro-4-benzazecine-3,8-dione is manufactured.

**19.** A process in accordance with claim 1, characterized in that 4-acetyl-1,2,4,5,6,7-hexahydro-12-methoxy-4-benzazecine-3,8-dione is manufactured.

**20.** A process for the preparation of benzoquinoline derivatives of general formula II defined in claim 1, characterized by

a) reducing a benzoquinolinone derivative of the general formula

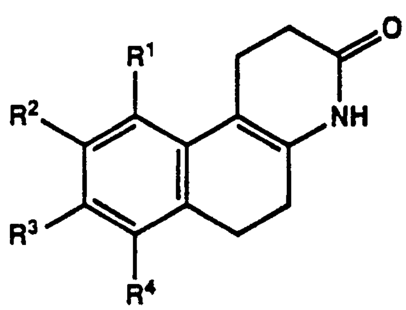

IV

wherein $R^1$, $R^2$, $R^3$ and $R^4$ have the significance given in claim 1,

or

b) reacting a compound of the general formula

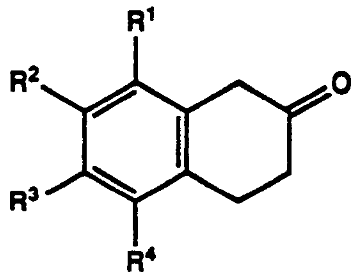

V

wherein $R^1$, $R^2$, $R^3$ and $R^4$ have the significance given in claim 1,

in the presence of a strong base with a compound of the general formula

$X-(CH_2)_3-NH_2$ VI

wherein X signifies a leaving group,
and, if desired, N-substituting the obtained compound of general formula II in which $R^5$ signifies hydrogen by acetyl, propionyl, benzoyl, chlorobenzoyl, methoxybenzoyl or phenylacetyl.

**21.** A process in accordance with claim 20 characterized in that 4-acetyl-8-chloro-1,2,3,4,5,6-hexahydrobenzo[f]quinoline is prepared.

**22.** A process in accordance with claim 20 characterized in that 4-acetyl-10-chloro-1,2,3,4,5,6-hexahydrobenzo[f]quinoline is prepared.

**23.** A process in accordance with claim 20 characterized in that 8-chloro-4-(p-chlorobenzoyl)-1,2,4,5,6-hexahydrobenzo-[f]quinoline is prepared.

**24.** The use of compounds of general formula 1 defined in claim 1 and of 4-acetyl-8-chloro-1,2,3,4,5,6-hexahydrobenzo-[f]quinoline, 4-acetyl-10-chloro-1,2,3,4,5,6-hexahydrobenzo-[f]quinoline and 8-chloro-4-(p-chlorobenzoyl)-1,2,3,4,5,6-hexahydrobenzo[f]quinoline for the manufacture of medicaments for the control or prevention of cerebral insufficiency or for the improvement of cognitive functions.

**Claims for the following Contracting State : GR**

**1.** A process for the manufacture of benzazecine derivatives of the general formula

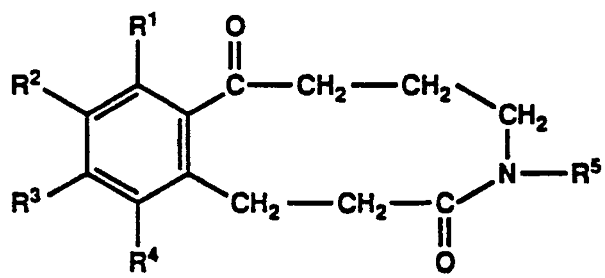

I

wherein $R^1$ and $R^2$ each signify hydrogen or chlorine, $R^3$ signifies hydrogen, fluorine, chlorine, bromine or methoxy, $R^4$ signifies hydrogen, chlorine or methoxy and $R^5$ signifies hydrogen, acetyl, propionyl, benzoyl, chlorobenzoyl, methoxybenzoyl or phenylacetyl, with the proviso that 2 or 3 of the symbols $R^1$ to $R^4$ signify hydrogen,
characterized by
a) oxidizing a benzoquinoline derivative of the general formula

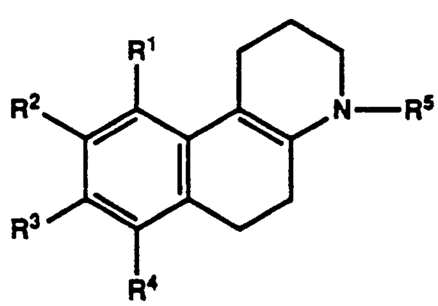

II

wherein $R^1$, $R^2$, $R^3$, $R^4$ and $R^5$ have the above significance,
or
b) removing the group denoted by Z from a benzazecine derivative of the general formula

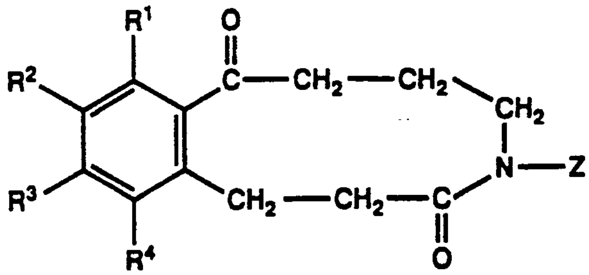

III

wherein $R^1$, $R^2$, $R^3$, and $R^4$ have the above significance and Z signifies a cleavable group.

2. A process in accordance with claim 1, characterized in that $R^1$ signifies chlorine and $R^2$, $R^3$ and $R^4$ each signify hydrogen.

3. A process in accordance with claim 1, characterized in that $R^2$ signifies chlorine and $R^1$, $R^3$ and $R^4$ each signify hydrogen.

4. A process in accordance with claim 1, characterized in that $R^3$ signifies fluorine, chlorine, bromine or methoxy and $R^1$, $R^2$ and $R^4$ each signify hydrogen.

5. A process in accordance with claim 1, characterized in that $R^4$ signifies chlorine or methoxy and $R^1$, $R^2$ and $R^3$ each signify hydrogen.

6. A process in accordance with claim 1, characterized in that $R^1$ and $R^3$ each signify chlorine and $R^2$ and $R^4$ each signify hydrogen.

7. A process in accordance with any one of claims 1 to 6, characterized in that $R^5$ signifies acetyl.

8. A process in accordance with claim 1, characterized in that 4-acetyl-9-chloro-1,2,4,5,6,7-hexahydro-4-benzazecine-3,8-dione is manufactured.

9. A process in accordance with claim 1, characterized in that 4-acetyl-11-chloro-1,2,4,5,6,7-hexahydro-4-benzazecine-3,8-dione is manufactured.

10. A process in accordance with claim 1, characterized in that 4-acetyl-11-fluoro-1,2,4,5,6,7-hexahydro-4-benzazecine-3,8-dione is manufactured.

11. A process in accordance with claim 1, characterized in that 4-acetyl-9,11-dichloro-1,2,4,5,6,7-hexahydro-4-benzazecine-3,8-dione is manufactured.

12. A process in accordance with claim 1, characterized in that 4-acetyl-11-methoxy-1,2,4,5,6,7-hexahydro-4-benzazecine-3,8-dione is manufactured.

13. A process in accordance with claim 1, characterized in that 11-chloro-1,2,4,5,6,7-hexahydro-4-propionyl-4-benzazecine-3,8-dione is manufactured.

14. A process in accordance with claim 1, characterized in that 4-benzoyl-11-chloro-1,2,4,5,6,7-hexahydro-4-benzazecine-3,8-dione is manufactured

15. A process in accordance with claim 1, characterized in that 4-(o-methoxybenzoyl)-11-chloro-1,2,4,5,6,7-hexahydro-4-benzazecine-3,8-dione is manufactured.

16. A process in accordance with claim 1, characterized in that 4-(m-methoxybenzoyl)-11-chloro-1,2,4,5,6,7-hexahydro-4-benzazecine-3,8-dione is manufactured.

17. A process in accordance with claim 1, characterized in that 4-(p-methoxybenzoyl)-11-chloro-1,2,4,5,6,7-hexahydro-4-benzazecine-3,8-dione is manufactured.

**18.** A process in accordance with claim 1, characterized in that 11-chloro-4-(p-chlorobenzoyl)-1,2,4,5,6,7-hexahydro-4-benzazecine-3,8-dione is manufactured.

**19.** A process in accordance with claim 1, characterized in that 4-acetyl-1,2,4,5,6,7-hexahydro-12-methoxy-4-benzazecine-3,8-dione is manufactured.

**20.** A process for the preparation of benzoquinoline derivatives of general formula II defined in claim 1, characterized by

a) reducing a benzoquinolinone derivative of the general formula

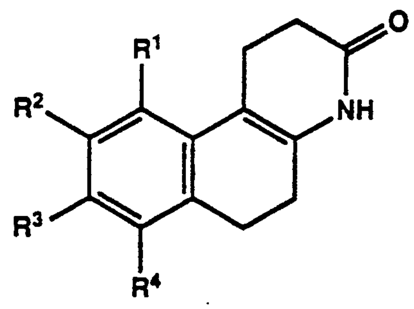

IV

wherein $R^1$, $R^2$, $R^3$ and $R^4$ have the significance given in claim 1,

or

b) reacting a compound of the general formula

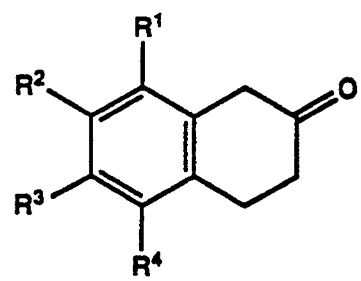

V

wherein $R^1$, $R^2$, $R^3$ and $R^4$ have the significance given in claim 1,

in the presence of a strong base with a compound of the general formula

$X-(CH_2)_3-NH_2$ VI

wherein X signifies a leaving group,

and, if desired, N-substituting the obtained compound of general formula II in which $R^5$ signifies hydrogen by acetyl, propionyl, benzoyl, chlorobenzoyl, methoxybenzoyl or phenylacetyl.

**21.** A process in accordance with claim 20 characterized in that 4-acetyl-8-chloro-1,2,3,4,5,6-hexahydrobenzo[f]quinoline is prepared.

**22.** A process in accordance with claim 20 characterized in that 4-acetyl-10-chloro-1,2,3,4,5,6-hexahydrobenzo[f]quinoline is prepared.

**23.** A process in accordance with claim 20 characterized in that 8-chloro-4-(p-chlorobenzoyl)-1,2,3,4,5,6-hexahydro-benzo[f]quinoline is prepared.

**24.** The use of compounds of general formula 1 defined in claim 1 and of 4-acetyl-8-chloro-1,2,3,4,5,6-hexahydrobenzo[f]quinoline, 4-acetyl-10-chloro-1,2,3,4,5,6-hexahydrobenzo[f]quinoline and 8-chloro-4-(p-chlorobenzoyl)-1,2,3,4,5,6-hexahydrobenzo[f]quinoline for the manufacture of medicaments for the

control or prevention of cerebral insufficiency or for the improvement of cognitive functions.

**25.** Benzazecine derivatives of the general formula

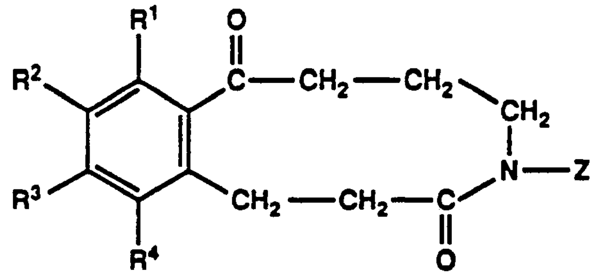

III

wherein $R^1$, $R^2$, $R^3$ and $R^4$ have the significance given in claim 1 and Z signifies a cleavable group, but not acetyl, propionyl, benzoyl, chlorobenzoyl, methoxybenzoyl or phenylacetyl.

**26.** Benzoquinoline derivatives of the general formula

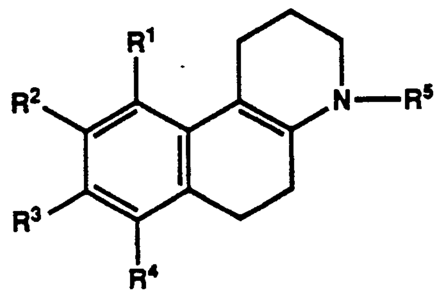

II

wherein $R^1$, $R^2$, $R^3$, $R^4$ and $R^5$ have the significance given in claim 1, but not 4-acetyl-8-chloro-1,2,3,4,5,6-hexahydrobenzo-[f]quinoline, 4-acetyl-10-chloro-1,2,3,4,5,6-hexahydrobenzo[f]quinoline and 8-chloro-4-(p-chlorobenzoyl)-1,2,3,4,5,6-hexahydrobenzo[f]quinoline.

**Revendications**

**Revendications pour les Etats contractants suivants : BE, CH, DE, FR, IT, LI, LU, NL, SE**

**1.** Dérivés de la benzazécine de formule générale

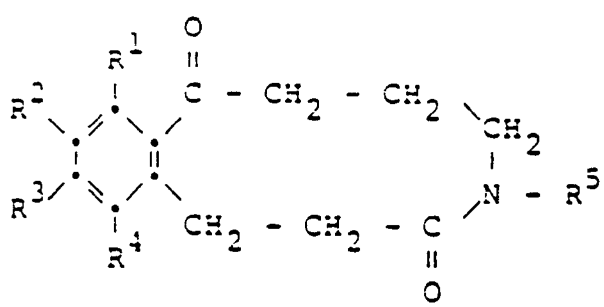

I

dans laquelle $R^1$ et $R^2$ représentent chacun l'hydrogène ou le chlore, $R^3$ représente l'hydrogène, le fluor, le chlore, le brome ou un groupe méthoxy, $R^4$ représente l'hydrogène, le chlore ou un groupe méthoxy et $R^5$ représente l'hydrogène, un groupe acétyle, propionyle, benzoyle, chlorobenzoyle, méthoxybenzoyle ou phénylacétyle, sous réserve que deux ou trois des symboles $R^1$ à $R^4$ représentent l'hydrogène.

**2.** Dérivés de la benzazécine selon revendication 1, dans lesquels $R^1$ représente le chlore et $R^2$, $R^3$ et $R^4$ représentent chacun l'hydrogène.

**3.** Dérivés de la benzazécine selon la revendication 1, dans lesquels $R^2$ représente le chlore et $R^1$, $R^3$ et $R^4$ représentent chacun l'hydrogène.

**4.** Dérivés de la benzazécine selon la revendication 1, dans lesquels $R^3$ représente le fluor, le chlore, le brome ou un groupe méthoxy et $R^1$, $R^2$ et $R^4$ représentent chacun l'hydrogène.

**5.** Dérivés de la benzazécine selon la revendication 1, dans lesquels $R^4$ représente le chlore ou un groupe méthoxy et $R^1$, $R^2$ et $R^3$ représentent chacun l'hydrogène.

**6.** Dérivés de la benzazécine selon la revendication 1, dans lesquels $R^1$ et $R^3$ représentent chacun le chlore et $R^2$ et $R^4$ représentent chacun l'hydrogène.

**7.** Dérivés de la benzazécine selon l'une des revendications 1 à 6, dans lesquels $R^5$ représente un groupe acétyle.

**8.** La 4-acétyl-9-chloro-1,2,4,5,6,7-hexahydro4-benzazécine-3,8-dione.

**9.** La 4-acétyl-11-chloro-1,2,4,5,6,7-hexahydro4-benzazécine-3,8-dione.

**10.** La 4-acétyl-11-fluoro-1,2,4,5,6,7-hexahydro4-benzazécine-3,8-dione.

**11.** La 4-acétyl-9,11-dichloro-1,2,4,5,6,7-hexahydro-4-benzazécine-3,8-dione.

**12.** La 4-acétyl-11-méthoxy-1,2,4,5,6,7-hexahydro4-benzazécine-3,8-dione.

**13.** La 11-chloro-1,2,4,5,6,7-hexahydro-4-propionyl-4-benzazécine-3,8-dione.

**14.** La 4-benzoyl-11-chloro-1,2,4,5,6,7-hexahydro4-benzazécine-3,8-dione.

**15.** La 4-(o-méthoxybenzoyl)-11-chloro-1,2,4,5,6,7-hexahydro-4-benzazécine-3,8-dione.

**16.** La 4-(m-méthoxybenzoyl)-11-chloro-1,2,4,5,6,7-hexahydro-4-benzazécine-3,8-dione.

**17.** La 4-(p-méthoxybenzoyl)-11-chloro-1,2,4,5,6,7-hexahydro-4-benzazécine-3,8-dione.

**18.** La 11-chloro-4-(p-chlorobenzoyl)-1,2,4,5,6,7-hexahydro-4-benzazécine-3,8-dione.

**19.** La 4-acétyl-1,2,4,5,6,7-hexahydro-12-méthoxy-4-benzazécine-3,8-dione.

**20.** La 9-chloro-1,2,4,5,6,7-hexahydro-4-benzazécine-3,8-dione ;
la 4-acétyl-10-chloro-1,2,4,5,6,7-hexahydro-4-benzazécine-3,8-dione ;
la 11-chloro-1,2,4,5,6,7-hexahydro-4-(phénylacétyl)-4-benzazécine-3,8-dione ;
la 11-chloro-1,2,4,5,6,7-hexahydro-4-benzazécine-3,8-dione ;
la 4-acétyl-11-bromo-1,2,4,5,6,7-hexahydro-4-benzazécine-3,8-dione ; et
la 4-acétyl-12-chloro-1,2,4,5,6,7-hexahydro-4-benzazécine-3,8-dione.

**21.** Dérivés de la benzoquinoléine de formule générale

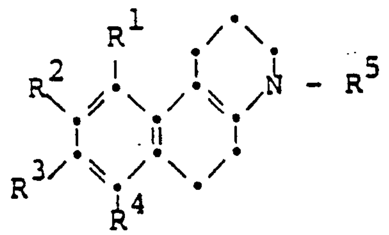

II

dans laquelle $R^1$, $R^2$, $R^3$ $R^4$ et $R^5$ ont les significations indiquées dans la revendication 1.

**22.** La 4-acétyl-8-chloro-1,2,3,4,5,6-hexahydrobenzo[f]quinoléine.

**23.** La 4-acétyl-10-chloro-1,2,3,4,5,6-hexahydrobenzo[f]quinoléine.

**24.** La 8-chloro-4-(p-chlorobenzoyl)-1,2,3,4,5,6-hexahydrobenzo[f]quinoléine.

**25.** Dérivés de la benzazécine de formule générale

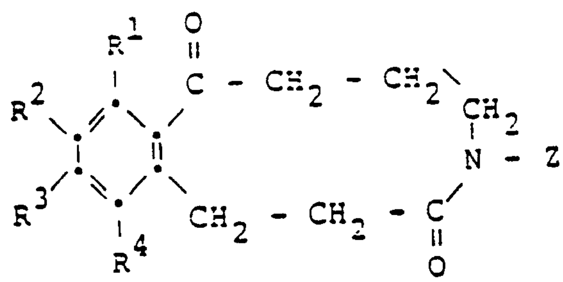

III

dans laquelle $R^1$, $R^2$, $R^3$ et $R^4$ ont les significations indiquées dans la revendication 1, et Z représente un groupe éliminable, à l'exception des groupes acétyle, propionyle, benzoyle, chlorobenzoyle, méthoxybenzoyle ou phénylacétyle.

**26.** Composés selon l'une des revendications 1 à 20 et 22 à 24, pour l'utilisation en tant que substances actives thérapeutiques.

**27.** Composés selon l'une des revendications 1 à 20 et 22 à 24, pour l'utilisation en tant que substances actives thérapeutiques agissant contre l'insuffisance cérébrale et améliorant les fonctions cognitives.

**28.** Procédé de préparation des dérivés de la benzazécine selon l'une des revendications 1 à 20, caractérisé en ce que :

a) on oxyde un dérivé de la benzoquinoléine de formule générale

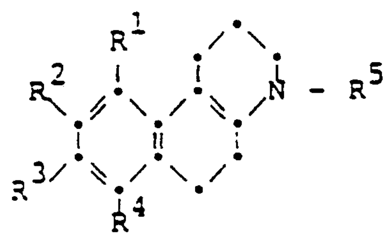

II

dans laquelle $R^1$, $R^2$, $R^3$, $R^4$ et $R^5$ ont les significations indiquées dans la revendication 1, ou bien

b) partant d'un dérivé de la benzazécine de formule générale

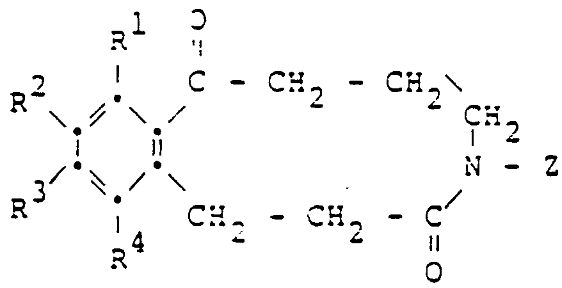

III

dans laquelle $R^1$, $R^2$, $R^3$ et $R^4$ ont les significations indiquées dans la revendication 1 et Z représente un groupe éliminable,
on élimine ce dernier groupe.

29. Procédé de préparation des dérivés de la benzoquinoléine selon l'une des revendications 21 à 24, caractérisé en ce que :
a) on réduit un dérivé de la benzoquinoléinone de formule générale

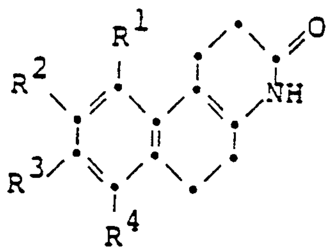

IV

dans laquelle $R^1$, $R^2$, $R^3$ et $R^4$ ont les significations indiquées dans la revendication 1, ou bien
b) on fait réagir un composé de formule générale

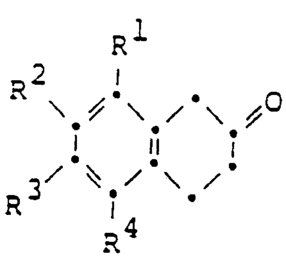

V

dans laquelle $R^1$, $R^2$, $R^3$ et $R^4$ ont les significations indiquées dans la revendication 1,
en présence d'une base forte, avec un composé de formule générale

$X-(CH_2)_3-NH_2$ VI

dans laquelle X représente un groupe éliminable, et si on le désire, on substitue à l'azote le composé ainsi obtenu répondant à la formule générale II dans laquelle $R^5$ représente l'hydrogène, par un groupe acétyle, propionyle, benzoyle, chlorobenzoyle, méthoxybenzoyle ou phénylacétyle.

30. Médicament contenant un composé selon l'une des revendications 1 à 20 et 22 à 24, et un excipient inerte du point de vue thérapeutique.

31. Médicament agissant contre l'insuffisance cérébrale et améliorant les fonctions cognitives, contenant un composé selon l'une des revendications 1 à 20 et 22 à 24 et un excipient inerte du point de vue

thérapeutique.

**32.** Composés selon l'une des revendications 1 à 20 et 22 à 24, pour le traitement ou la prévention de maladies ou l'amélioration de la santé.

**33.** Composés selon l'une des revendications 1 à 20 et 22 à 24, pour le traitement et la prévention de l'insuffisance cérébrale ou l'amélioration des fonctions cognitives.

**34.** Utilisation des composés selon l'une des revendications 1 à 20 et 22 à 24, pour la préparation de médicaments servant au traitement ou à la prévention de l'insuffisance cérébrale ou à l'amélioration des fonctions cognitives.

**Revendications pour les Etats contractants suivantes : AT, ES**

**1.** Procédé de préparation de dérivés de la benzazécine de formule générale

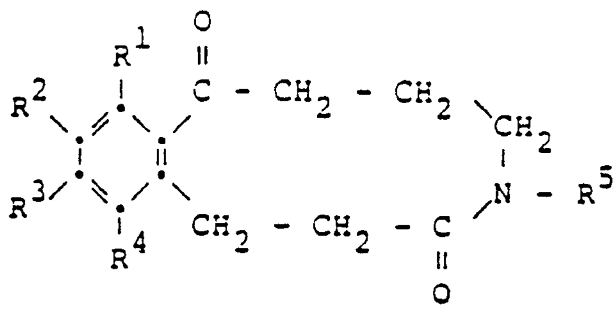

I

dans laquelle $R^1$ et $R^2$ représentent chacun l'hydrogène ou le chlore, $R^3$ représente l'hydrogène, le fluor, le chlore, le brome ou un groupe méthoxy, $R^4$ représente l'hydrogène, le chlore ou un groupe méthoxy et $R^5$ l'hydrogène ou un groupe acétyle, propionyle, benzoyle, chlorobenzoyle, méthoxybenzoyle ou phénylacétyle, sous réserve que deux ou trois des symboles $R^1$ à $R^4$ représentent l'hydrogène, caractérisé en ce que :

a) on oxyde un dérivé de la benzoquinoléine de formule générale

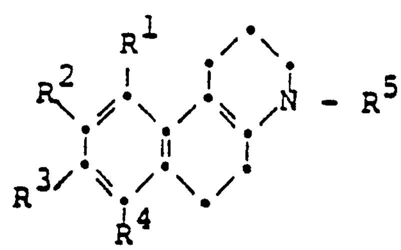

II

dans laquelle $R^1$, $R^2$, $R^3$, $R^4$ et $R^5$ ont les significations indiquées ci-dessus, ou bien

b) partant d'un dérivé de la benzazécine de formule générale

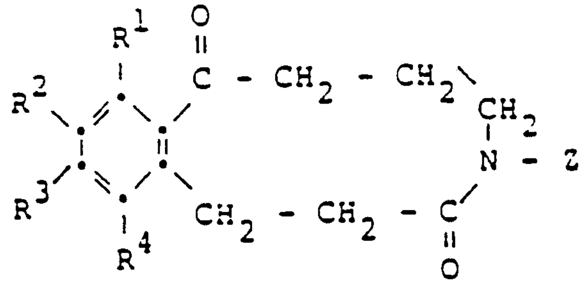

III

dans laquelle $R^1$, $R^2$, $R^3$ et $R^4$ ont les significations indiquées ci-dessus et Z représente un groupe éliminable, on élimine ce dernier groupe.

2. Procédé selon la revendioation 1, caractérisé en ce que $R^1$ représente le chlore et $R^2$, $R^3$ et $R^4$ représentent chacun l'hydrogène.

3. Procédé selon la revendication 1, caractérisé en ce que $R^2$ représente le chlore et $R^1$, $R^3$ et $R^4$ représentent chacun l'hydrogène.

4. Procédé selon la revendication 1, caractérisé en ce que $R^3$ représente le fluor, le chlore, le brome ou un groupe méthoxy et $R^1$, $R^2$ et $R^4$ représentent chacun l'hydrogène.

5. Procédé selon la revendication 1, caractérisé en ce que $R^4$ représente le chlore ou un groupe méthoxy et $R^1$, $R^2$ et $R^3$ représentent chacun l'hydrogène.

6. Procédé selon la revendication 1, caractérisé en ce que $R^1$ et $R^3$ représentent chacun le chlore et $R^2$ et $R^4$ chacun l'hydrogène.

7. Procédé selon l'une des revendications 1 à 6, caractérisé en ce que $R^5$ représente un groupe acétyle.

8. Procédé selon la revendication 1, caractérisé en ce que l'on prépare la 4-acétyl-9-chloro-1,2,4,5,6,7-hexahydro-4-benzazécine-3,8-dione.

9. Procédé selon la revendication 1, caractérisé en ce que l'on prépare la 4-acétyl-11-chloro-1,2,4,5,6,7-hexahydro-4-benzazécine-3,8-dione.

10. Procédé selon la revendication 1, caractérisé en ce que l'on prépare la 4-acétyl-11-fluoro-1,2,4,5,6,7-hexahydro-4-benzazécine-3,8-dione.

11. Procédé selon la revendication 1, caractérisé en ce que l'on prépare la 4-acétyl-9,11-dichloro-1,2,4,5,6,7-hexahydro-4-benzazécine-3,8-dione.

12. Procédé selon la revendication 1, caractérisé en ce que l'on prépare la 4-acétyl-11-méthoxy-1,2,4,5,6,7-hexahydro-4-benzazécine-3,8-dione.

13. Procédé selon la revendication 1, caractérisé en ce que l'on prépare la 11-chloro-1,2,4,5,6,7-hexahydro-4-propinoyl-4-benzazécine-3,8-dione.

14. Procédé selon la revendication 1, caractérisé en ce que l'on prépare la 4-benzoyl-11-chloro-1,2,4,5,6,7-hexahydro-4-benzazécine-3,8-dione.

15. Procédé selon la revendication 1, caractérisé en ce que l'on prépare la 4-(o-méthoxybenzoyl)-11-chloro-1,2,4,5,6,7-hexahydro-4-benzazécine-3,8-dione.

16. Procédé selon la revendication 1, caractérisé en ce que l'on prépare la 4-(m-méthoxybenzoyl)-11-chloro-1,2,4,5,6,7-hexahydro-4-benzazécine-3,8-dione.

17. Procédé selon la revendication 1, caractérisé en ce que l'on prépare la 4-(p-méthoxybenzoyl)-11-chloro-1,2,4,5,6,7-hexahydro-4-benzazécine-3,8-dione.

18. Procédé selon la revendication 1, caractérisé en ce que l'on prépare la 11-chloro-4-(p-chlorobenzoyl)-1,2,4,5,6,7-hexahydro-4-benzazécine-3,8-dione.

19. Procédé selon la revendication 1, caractérisé en ce que l'on prépare la 4-acétyl-1,2,4,5,6,7-hexahydro-12-méthoxy-4-benzazécine-3,8-dione.

20. Procédé de préparation des dérivés de la benzoquinoléine de formule générale II de la revendication 1, caractérisé en ce que :

a) on réduit un dérivé de la benzoquinoléinone de formule générale

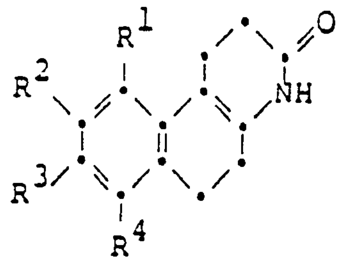

IV

dans laquelle $R^1$, $R^2$, $R^3$ et $R^4$ ont les significations indiquées dans la revendication 1, ou bien
b) on fait réagir un composé de formule générale

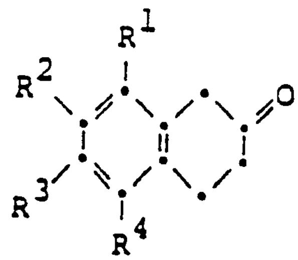

V

dans laquelle $R^1$, $R^2$, $R^3$ et $R^4$ ont les significations indiquées dans la revendication 1, en présence d'une base forte, avec un composé de formule générale

$X-(CH_2)_3-NH_2$ VI

dans laquelle X représente un groupe éliminable, et si on le désire, on substitue à l'azote le composé ainsi obtenu répondant à la formule générale II dans laquelle $R^5$ représente l'hydrogène, par un groupe acétyle, propionyle, benzoyle, chlorobenzoyle, méthoxybenzoyle ou phénylacétyle.

**21.** Procédé selon la revendication 20, caractérisé en ce que l'on prépare la 4-acétyl-8-chloro-1,2,3, 4,5,6-hexahydrobenzo[f]quinoléine.

**22.** Procédé selon la revendication 20, caractérisé en ce que l'on prépare la 4-acétyl-10-chloro-1,2,3, 4,5,6-hexahydrobenzo[f]quinoléine.

**23.** Procédé selon la revendication 20, caractérisé en ce que l'on prépare la 8-chloro-4-(p-chloro-benzoyl)-1,2,3,4,5,6-hexahydrobenzo[f]quinoléine.

**24.** Utilisation des composés de formule générale I de la revendication 1, ainsi que de la 4-acétyl-8-chloro-1,2,3,4,5,6-hexahydrobenzo[f]quinoléine, de la 4-acétyl-10-chloro-1,2,3,4,5,6-hexahydrobenzo[f]-quinoleine et de la 8-chloro-4-(p-chlorobenzoyl)-1,2,3,4,5,6-hexahydrobenzo[f]quinoléine, pour la préparation de médicaments servant au traitement ou à la prévention de l'insuffisance cérébrale et à l'amélioration des fonctions cognitives.

**Revendications pour l'Etat contractant suivant : GR**

**1.** Procédé de préparation de dérivés de la benzazécine de formule générale

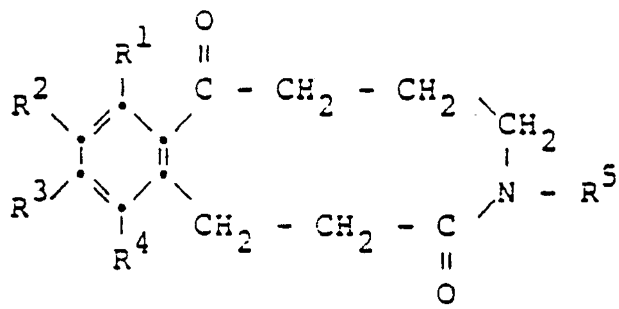

I

dans laquelle $R^1$ et $R^2$ représentent chacun l'hydrogène ou le chlore, $R^3$ représente l'hydrogène, le fluor, le chlore, le brome ou un groupe méthoxy, $R^4$ représente l'hydrogène, le chlore ou un groupe méthoxy et $R^5$ représente l'hydrogène ou un groupe acétyle, propionyle, benzoyle, chlorobenzoyle, méthoxybenzoyle ou phénylacétyle, sous réserve que deux ou trois des symboles $R^1$ à $R^4$ représentent l'hydrogène, caractérisé en ce que :

a) on oxyde un dérivé de la benzoquinoléine de formule générale

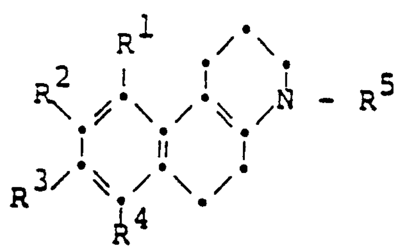

II

dans laquelle $R^1$, $R^2$, $R^3$, $R^4$ et $R^5$ ont les significations indiquées ci-dessus, ou bien

b) partant d'un dérivé de la benzazécine de formule générale

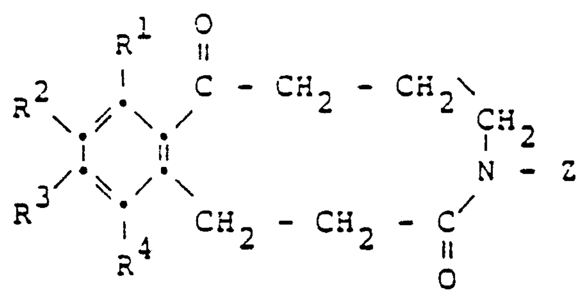

III

dans laquelle $R^1$, $R^2$, $R^3$ et $R^4$ ont les significations indiquées ci-dessus et Z représente un groupe éliminable, on élimine ce dernier groupe.

2. Procédé selon la revendication 1, caractérisé en ce que $R^1$ représente le chlore et $R^2$, $R^3$ et $R^4$ représentent chacun l'hydrogène.

3. Procédé selon la revendication 1, caractérisé en ce que $R^2$ représente le chlore et $R^1$, $R^3$ et $R^4$ représentent chacun l'hydrogène.

4. Procédé selon la revendication 1, caractérisé en ce que $R^3$ représente le fluor, le chlore, le brome ou un groupe méthoxy et $R^1$, $R^2$ et $R^4$ représentent chacun l'hydrogène.

5. Procédé selon la revendication 1, caractérisé en ce que $R^4$ représente le chlore ou un groupe méthoxy et $R^1$, $R^2$ et $R^3$ représentent chacun l'hydrogène.

6. Procédé selon la revendication 1, caractérisé en ce que $R^1$ et $R^3$ représentent chacun le chlore et $R^2$

et $R^4$ chacun l'hydrogène.

7. Procédé selon l'une des revendications 1 à 6, caractérisé en ce que $R^5$ représente un groupe acétyle.

8. Procédé selon la revendication 1, caractérisé en ce que l'on prépare la 4-acétyl-9-chloro-1,2,4,5,6,7-hexahydro-4-benzazécine-3,8-dione.

9. Procédé selon la revendication 1, caractérisé en ce que l'on prépare la 4-acétyl-11-chloro-1,2,4,5,6,7-hexahydro-4-benzazécine-3,8-dione.

10. Procédé selon la revendication 1,caractérisé en ce que l'on prépare la 4-acétyl-11-fluoro-1,2,4,5,6,7-hexahydro-4-benzazécine-3,8-dione.

11. Procédé selon la revendication 1, caractérisé en ce que l'on prépare la 4-acétyl-9,11-dichloro-1,2,4,5,6,7-hexahydro-4-benzazécine-3,8-dione.

12. Procédé selon la revendication 1, caractérisé en ce que l'on prépare la 4-acétyl-11-méthoxy-1,2,4,5,6,7-hexahydro-4-benzazécine-3,8-dione.

13. Procédé selon la revendication 1, caractérisé en ce que l'on prépare la 11-chloro-1,2,4,5,6,7-hexahydro-4-propionyl-4-benzazécine-3,8-dione.

14. Procédé selon la revendication 1, caractérisé en ce que l'on prépare la 4-benzoyl-11-chloro-1,2,4,5,6,7-hexahydro-4-benzazécine-3,8-dione.

15. Procédé selon la revendication 1, caractérisé en ce que l'on prépare la 4-(o-méthoxybenzoyl)-11-chloro-1,2,4,5,6,7-hexahydro-4-benzazécine-3,8-dione.

16. Procédé selon la revendication 1, caractérisé en ce que l'on prépare la 4-(m-méthoxybenzoyl)-11-chloro-1,2,4,5,6,7-hexahydro-4-benzazécine-3,8-dione.

17. Procédé selon la revendication 1, caractérisé en ce que l'on prépare la 4-(p-méthoxybenzoyl)-11-chloro-1,2,4,5,6,7-hexahydro-4-benzazécine-3,8-dione.

18. Procédé selon la revendication 1, caractérisé en ce que l'on prépare la 11-chloro-4-(p-chlorobenzoyl)-1,2,4,5,6,7-hexahydro-4-benzazécine-3,8-dione.

19. Procédé selon la revendication 1, caractérisé en ce que l'on prépare la 4-acétyl-1,2,4,5,6,7-hexahydro-12-méthoxy-4-benzazécine-3,8-dione.

20. Procédé de préparation des dérivés de la benzoquinoléine de formule générale II de la revendication 1, caractérisé en ce que :

a) on réduit un dérivé de la benzoquinoléinone de formule générale

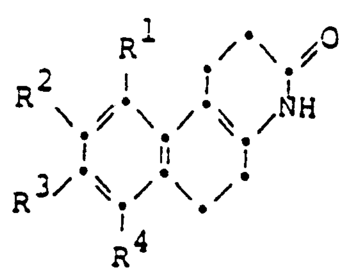

IV

dans laquelle $R^1$, $R^2$, $R^3$ et $R^4$ ont les significations indiquées dans la revendication 1, ou bien

b) on fait réagir un composé de formule générale

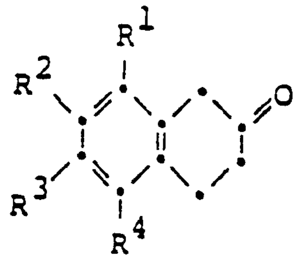

V

dans laquelle $R^1$, $R^2$, $R^3$ et $R^4$ ont les significations indiquées dans la revendication 1, en présence d'une base forte, avec un composé de formule générale

$X-(CH_2)_3-NH_2$ VI

dans laquelle X représente un groupe éliminable, et si on le désire, on substitue à l'azote le composé ainsi obtenu répondant à la formule générale II dans laquelle $R^5$ représente l'hydrogène, par un groupe acétyle, propionyle, benzoyle, chlorobenzoyle, méthoxybenzoyle ou phénylacétyle.

**21.** Procédé selon la revendication 20, caractérisé en ce que l'on prépare la 4-acétyl-8-chloro-1,2,3,4,5,6-hexahydrobenzo[f]quinoléine.

**22.** Procédé selon la revendication 20, caractérisé en ce que l'on prépare la 4-acétyl-10-chloro-1,2,3,4,5,6-hexahydrobenzo[f]quinoléine.

**23.** Procédé selon la revendication 20, caractérisé en ce que l'on prépare la 8-chloro-4-(p-chloro-benzoyl)-1,2,3,4,5,6-hexahydrobenzo[f]quinoléine.

**24.** Utilisation des composés de formule générale I de la revendication 1, ainsi que de la 4-acétyl-8-chloro-1,2,3,4,5,6-hexahydrobenzo[f]quinoléine, de la 4-acétyl-10-chloro-1,2,3,4,5,6-hexahydrobenzo[f]-quinoléine et de la 8-chloro-4-(p-chlorobenzoyl)-1,2,3,4,5,6-hexahydrobenzo[f]quinoléine, pour la préparation de médicaments servant au traitement ou à la prévention de l'insuffisance cérébrale et à l'amélioration des fonctions cognitives.

**25.** Dérivés de la benzazécine de formule générale

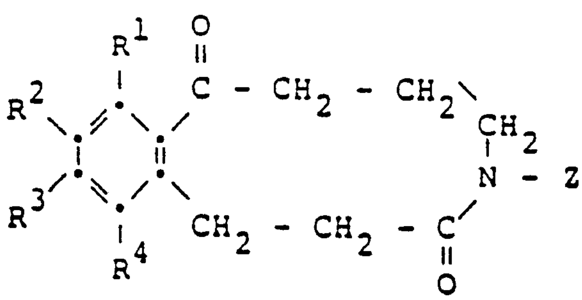

III

dans laquelle $R^1$, $R^2$, $R^3$ et $R^4$ ont les significations indiquées dans la revendication 1 et Z représente un groupe éliminable, à l'exception des groupes acétyle, propionyle, benzoyle, chlorobenzoyle, méthoxybenzoyle ou phénylacétyle.

**26.** Dérivés de la benzoquinoléine de formule générale

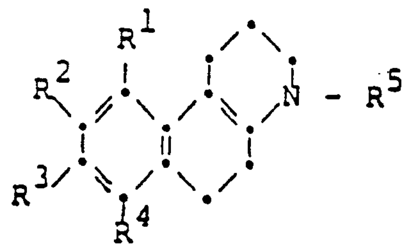

II

dans laquelle $R^1$, $R^2$, $R^3$, $R^4$ et $R^5$ ont les significations indiquées dans la revendication 1, à l'exception de la 4-acétyl-8-chloro-1,2,3,4,5,6-hexahydrobenzo[f]quinoléine, de la 4-acétyl-10-chloro-1,2,3,4,5,6-hexahydrobenzo[f]quinoléine et de la 8-chloro-4-(p-chlorobenzoyl)-1,2,3,4,5,6-hexahydrobenzo[f]-quinoléine.